# EUROPEAN PATENT APPLICATION

(11) **EP 1 959 020 A1**
(43) Date of publication of application: **20.08.2008**
(21) Application number: 06823559.7
(22) Date of filing: 01.12.2006
(51) Int. Cl.: C12P 13/00, C12N 15/09

(54) **PROCESS FOR PRODUCTION OF CERAMIDE USING TRANSFORMED YEAST**

(30) Priority: 05.12.2005 JP 2005351366
(71) Applicant: Suntory Limited, Osaka-shi Osaka 530-8203 (JP); Hiroshima University, Higashi-Hiroshima-shi Hiroshima 739-8511 (JP)
(72) Inventor: KODAMA, Yukiko, Mishima-gun, Osaka 618-8503 (JP); NAGANO, Hideaki, Mishima-gun, Osaka 618-8503 (JP); FUNATO, Koichi, Higashi-Hiroshima-shi, Hiroshima 739-8528 (JP)
(74) Representative: Vossius & Partner
(86) International application number: PCT/JP2006/324080
(87) International publication number: WO 2007/066595

(57) **Abstract**

The present invention provides methods for producing human ceramide in a yeast cell.

The methods of the present invention comprise:
1) introducing the sphingoid Δ4-desaturase gene (DES1) by transformation of the yeast cell; and
2) abolishing the expression of the yeast sphinganine C4-hydroxylase gene (SUR2) by transformation of the yeast cell.

SEQUENCE LISTING
<110> Suntory Limited
<120> Method for producing ceramide using transformed yeast
<130> S-38-359
<160> 32

<210> 1
<211> 972
<212> DNA
<213> Homo sapiens
<400> 1
atggggagcc gcgtctcgcg ggaagacttc gagtgggtct acaccgacca gccgcacgcc 60
gaccggcgcc gggagatcct ggcaaagtat ccagagataa agtccttgat gaaacctgat 120
cccaatttga tatggattat aattatgatg gttctcaccc agttgggtgc attttacata 180
gtaaaagact tggactggaa atgggtcata tttggggcct atgcgtttgg cagttgcatt 240
aaccactcaa tgactctggc tattcatgag attgcccaca atgctgcctt tggcaactgc 300
aaagcaatgt ggaatcgctg gtttggaatg tttgctaatc ttcctattgg gattccatat 360
tcaatttcct ttaagaggta tcacatggat catcatcggt accttggagc tgatggcgtc 420
gatgtagata ttcctaccga ttttgagggc tggttcttct gtaccgcttt cagaaagttt 480
atatgggtta ttcttcagcc tctcttttat gcctttcgac ctctgttcat caaccccaaa 540
ccaattacgt atctggaagt tatcaatacc gtggcacagg tcacttttga cattttaatt 600
tattactttt tgggaattaa atccttagtc tacatgttgg cagcatcttt acttggcctg 660
ggtttgcacc caatttctgg acattttata gctgagcatt acatgttctt aaagggtcat 720
gaaacttact catattatgg gcctctgaat ttacttacct tcaatgtggg ttatcataat 780
gaacatcatg atttccccaa cattcctgga aaaagtcttc cactggtgag gaaaatagca 840
gctgaatact atgacaacct ccctcactac aattcctgga taaaagtact gtatgatttt 900
gtgatggatg atacaataag tccctactca agaatgaaga ggcaccaaaa aggagagatg 960
gtgctggagt aa 972

<210> 2
<211> 323
<212> PRT
<213> Homo sapiens
<400> 2
Met Gly Ser Arg Val Ser Arg Glu Asp Phe Glu Trp Val Tyr Thr
1               5                   10                  15
Asp Gln Pro His Ala Asp Arg Arg Arg Glu Ile Leu Ala Lys Tyr
                20                  25                  30
Pro Glu Ile Lys Ser Leu Met Lys Pro Asp Pro Asn Leu Ile Trp
                35                  40                  45
Ile Ile Ile Met Met Val Leu Thr Gln Leu Gly Ala Phe Tyr Ile
                50                  55                  60
Val Lys Asp Leu Asp Trp Lys Trp Val Ile Phe Gly Ala Tyr Ala
                65                  70                  75
Phe Gly Ser Cys Ile Asn His Ser Met Thr Leu Ala Ile His Glu
                80                  85                  90
Ile Ala His Asn Ala Ala Phe Gly Asn Cys Lys Ala Met Trp Asn
                95                  100                 105
Arg Trp Phe Gly Met Phe Ala Asn Leu Pro Ile Gly Ile Pro Tyr
                110                 115                 120
Ser Ile Ser Phe Lys Arg Tyr His Met Asp His His Arg Tyr Leu
                125                 130                 135
Gly Ala Asp Gly Val Asp Val Asp Ile Pro Thr Asp Phe Glu Gly
                140                 145                 150
Trp Phe Phe Cys Thr Ala Phe Arg Lys Phe Ile Trp Val Ile Leu
                155                 160                 165
Gln Pro Leu Phe Tyr Ala Phe Arg Pro Leu Phe Ile Asn Pro Lys
                170                 175                 180
Pro Ile Thr Tyr Leu Glu Val Ile Asn Thr Val Ala Gln Val Thr
                185                 190                 195
Phe Asp Ile Leu Ile Tyr Tyr Phe Leu Gly Ile Lys Ser Leu Val
                200                 205                 210
Tyr Met Leu Ala Ala Ser Leu Leu Gly Leu Gly Leu His Pro Ile
                215                 220                 225
Ser Gly His Phe Ile Ala Glu His Tyr Met Phe Leu Lys Gly His
                230                 235                 240
Glu Thr Tyr Ser Tyr Tyr Gly Pro Leu Asn Leu Leu Thr Phe Asn
                245                 250                 255
Val Gly Tyr His Asn Glu His His Asp Phe Pro Asn Ile Pro Gly
                260                 265                 270
Lys Ser Leu Pro Leu Val Arg Lys Ile Ala Ala Glu Tyr Tyr Asp
                275                 280                 285
sn Leu Pro His Tyr Asn Ser Trp Ile Lys Val Leu Tyr Asp Phe
290 295 300
Val Met Asp Asp Thr Ile Ser Pro Tyr Ser Arg Met Lys Arg His
305 310 315

Gln Lys Gly Glu Met Val Leu Glu
                320         323

<210>  3
<211>  1434
<212>  DNA
<213>  Saccharomyces cerevisiae
<400>  3
atgtacaaca gaaaagacag agatgttcac gagaggaagg aagatggtca atctgagttt     60
gaagcactga acgggaccaa cgcaattatg tccgataata gtaaagcgta ttccataaag    120
tttctgacct tcaatacatg ggggttaaaa tacgtctcca aacaccgtaa agaaagactc    180
agagcaattg ctgataaatt ggcgggccac tcaatgctta cgccaatatc tgacgagttg    240
ttgcccaatg gtggagatag taatgaaaac gaagattacg acgtgattgc cttacaagaa    300
atctggtgtg tggaagactg gaagtatcta gcttctgcgt gtgcctccaa gtatccgtat    360
cagcgtttgt tccattctgg tattctgacg ggccctgggt tggccatact gtccaaggtc    420
ccgatagagt cgacctttct ttaccggttc ccgataaacg gtagaccgag tgcggtgttc    480
cgtggcgact ggtacgtagg gaaatctata gcaatcaccg tattgaacac aggaacccgc    540
cccattgcaa taatgaacag tcacatgcac gccccatacg ccaagcaggg tgatgccgcc    600
tacttgtgcc acagatcttg tcaggcctgg gatttcagca ggctcattaa gctttacagg    660
caggccggtt atgcggtgat tgtggtgggt gacttaaact ccagaccggg ctcactgccc    720
cacaaatttc tcacgcagga ggccggcctg gtcgactcct gggagcaatt gcatgggaag    780
caagacttgg cggtgatcgc tcgtctgtct ccattgcaac aattgcttaa gggctgtacc    840
acgtgcgatt cgctgctcaa cacatggagg gcccaaagac aacccgatga ggcatgcagg    900
ttggattatg ctcttatcga ccctgatttc ttgcaaacag tagacgcagg tgtcaggttc    960
actgaacgga tccctcacct ggactgcagt gtctctgacc attttgcata ctcatgcacc   1020
cttaacatcg tcccacaggg cacagagtcc cgtccatcca cctccgttaa gcgtgcgaag   1080
actcatgata gagagctgat cttgcagaga tactccaact acgaaaccat gatagaatgc   1140
atccacacgt acttgaagac agcccaaaga cagaaatttt tccgtggcct acatttctgg   1200
gcctcaatac ttctcctaat agcgtcgttg gtcgtgacaa cgtttactgc aaacaaggca   1260
ggctggtcct ccatcttctg ggtccttttc gctattgctg tctccatctc gggcaccatc   1320
gacggtgcca tctccttctt gtttggcagg tctgaaatca gagccctcat cgaagtcgaa   1380
caagaggttc tggacgcgga gcaccacctg caaactttct tgagcgagaa atga   1434

<210>  4
<211>  477
<212>  PRT
<213>  Saccharomyces cerevisiae
<400>  4
Met Tyr Asn Arg Lys Asp Arg Asp Val His Glu Arg Lys Glu Asp
  1               5                  10                  15
Gly Gln Ser Glu Phe Glu Ala Leu Asn Gly Thr Asn Ala Ile Met
                 20                  25                  30
Ser Asp Asn Ser Lys Ala Tyr Ser Ile Lys Phe Leu Thr Phe Asn
                 35                  40                  45
Thr Trp Gly Leu Lys Tyr Val Ser Lys His Arg Lys Glu Arg Leu
                 50                  55                  60
Arg Ala Ile Ala Asp Lys Leu Ala Gly His Ser Met Leu Thr Pro
                 65                  70                  75
Ile Ser Asp Glu Leu Leu Pro Asn Gly Gly Asp Ser Asn Glu Asn
                 80                  85                  90
Glu Asp Tyr Asp Val Ile Ala Leu Gln Glu Ile Trp Cys Val Glu
                 95                 100                 105
Asp Trp Lys Tyr Leu Ala Ser Ala Cys Ala Ser Lys Tyr Pro Tyr
                110                 115                 120
Gln Arg Leu Phe His Ser Gly Ile Leu Thr Gly Pro Gly Leu Ala
                125                 130                 135
Ile Leu Ser Lys Val Pro Ile Glu Ser Thr Phe Leu Tyr Arg Phe
                140                 145                 150
Pro Ile Asn Gly Arg Pro Ser Ala Val Phe Arg Gly Asp Trp Tyr
                155                 160                 165
Val Gly Lys Ser Ile Ala Ile Thr Val Leu Asn Thr Gly Thr Arg
                170                 175                 180
Pro Ile Ala Ile Met Asn Ser His Met His Ala Pro Tyr Ala Lys
                185                 190                 195
Gln Gly Asp Ala Ala Tyr Leu Cys His Arg Ser Cys Gln Ala Trp
                200                 205                 210
Asp Phe Ser Arg Leu Ile Lys Leu Tyr Arg Gln Ala Gly Tyr Ala
                215                 220                 225
Val Ile Val Val Gly Asp Leu Asn Ser Arg Pro Gly Ser Leu Pro
                230                 235                 240
His Lys Phe Leu Thr Gln Glu Ala Gly Leu Val Asp Ser Trp Glu
                245                 250                 255
Gln Leu His Gly Lys Gln Asp Leu Ala Val Ile Ala Arg Leu Ser
                260                 265                 270
Pro Leu Gln Gln Leu Leu Lys Gly Cys Thr Thr Cys Asp Ser Leu
                275                 280                 285

Leu Asn Thr Trp Arg Ala Gln Arg Gln Pro Asp Glu Ala Cys Arg
                290                 295                 300
Leu Asp Tyr Ala Leu Ile Asp Pro Asp Phe Leu Gln Thr Val Asp
                305                 310                 315
Ala Gly Val Arg Phe Thr Glu Arg Ile Pro His Leu Asp Cys Ser
                320                 325                 330
Val Ser Asp His Phe Ala Tyr Ser Cys Thr Leu Asn Ile Val Pro
                335                 340                 345
Gln Gly Thr Glu Ser Arg Pro Ser Thr Ser Val Lys Arg Ala Lys
                350                 355                 360
Thr His Asp Arg Glu Leu Ile Leu Gln Arg Tyr Ser Asn Tyr Glu
                365                 370                 375
Thr Met Ile Glu Cys Ile His Thr Tyr Leu Lys Thr Ala Gln Arg
                380                 385                 390
Gln Lys Phe Phe Arg Gly Leu His Phe Trp Ala Ser Ile Leu Leu
                395                 400                 405
Leu Ile Ala Ser Leu Val Val Thr Thr Phe Thr Ala Asn Lys Ala
                410                 415                 420
Gly Trp Ser Ser Ile Phe Trp Val Leu Phe Ala Ile Ala Val Ser
                425                 430                 435
Ile Ser Gly Thr Ile Asp Gly Ala Ile Ser Phe Leu Phe Gly Arg
                440                 445                 450
Ser Glu Ile Arg Ala Leu Ile Glu Val Glu Gln Glu Val Leu Asp
                455                 460                 465
Ala Glu His His Leu Gln Thr Phe Leu Ser Glu Lys
                470                 475     477

<210>  5
<211>  1050
<212>  DNA
<213>  Saccharomyces cerevisiae
<400>  5
atgaacgtaa catcgaatgc aactgcagcc ggttcctttc cactagcatt tggtctcaag    60
acctcatttg ggtttatgca ctatgccaag gcccctgcca ttaatttacg ccccaaggaa   120
tccttgctgc cggaaatgag tgatggtgtg ctggccttgg ttgcgccggt tgttgcctac   180
tgggcgttgt ctggtatatt ccatgtaata gacactttcc atctggctga gaagtacaga   240
attcatccga gcgaagaggt tgccaagagg aacaaggcgt cgagaatgca tgttttcctt   300
gaagtgattc tacaacatat catacagacc attgttggcc ttatctttat gcacttcgag   360
ccgatctaca tgactgggtt tgaagaaaat gccatgtgga agcttcgtgc agaccttcct   420
cggattattc cagatgccgc tatttattac ggctatatgt acggaatgtc cgctttgaag   480
atctttgcag gctttttatt cgttgataca tggcaatact tttgcatag attgatgcat    540
atgaataaga ccttatacaa atggttccac tctgttcatc atgaactata cgtgccatat   600
gcttacggtg ctcttttcaa caatcctgtt gagggcttct tgttagatac tttgggaacc   660
ggtattgcca tgacgttaac tcatttgact cacagagagc aaatcattct tttacctttt   720
gccaccatga agactgtcga tgaccactgt gggtatgctt tgccacttga cccattccaa   780
tggcttttcc ctaataacgc tgtctatcac gatatccacc accagcaatt tggtatcaag   840
acgaactttg ctcaaccatt tttcactttc tgggacaatt tgttccaaac taactttaaa   900
gggtttgaag aatatcaaaa gaagcaaaga cgtgtcacca tcgacaagta caaagagttt   960
ttgcaagaga gagaattgga aaagaaggag aaactcaaaa acttcaaagc tatgaatgct  1020
gctgaaaatg aagtaaagaa agagaaataa  1050

<210>  6
<211>  349
<212>  PRT
<213>  Saccharomyces cerevisiae
<400>  6
Met Asn Val Thr Ser Asn Ala Thr Ala Ala Gly Ser Phe Pro Leu
 1               5                  10                  15
Ala Phe Gly Leu Lys Thr Ser Phe Gly Phe Met His Tyr Ala Lys
                20                  25                  30
Ala Pro Ala Ile Asn Leu Arg Pro Lys Glu Ser Leu Leu Pro Glu
                35                  40                  45
Met Ser Asp Gly Val Leu Ala Leu Val Ala Pro Val Val Ala Tyr
                50                  55                  60
Trp Ala Leu Ser Gly Ile Phe His Val Ile Asp Thr Phe His Leu
                65                  70                  75
Ala Glu Lys Tyr Arg Ile His Pro Ser Glu Glu Val Ala Lys Arg
                80                  85                  90
Asn Lys Ala Ser Arg Met His Val Phe Leu Glu Val Ile Leu Gln
                95                 100                 105
His Ile Ile Gln Thr Ile Val Gly Leu Ile Phe Met His Phe Glu
               110                 115                 120
Pro Ile Tyr Met Thr Gly Phe Glu Glu Asn Ala Met Trp Lys Leu
               125                 130                 135
Arg Ala Asp Leu Pro Arg Ile Ile Pro Asp Ala Ala Ile Tyr Tyr
               140                 145                 150

Gly Tyr Met Tyr Gly Met Ser Ala Leu Lys Ile Phe Ala Gly Phe
                155                 160                 165
Leu Phe Val Asp Thr Trp Gln Tyr Phe Leu His Arg Leu Met His
                170                 175                 180
Met Asn Lys Thr Leu Tyr Lys Trp Phe His Ser Val His His Glu
                185                 190                 195
Leu Tyr Val Pro Tyr Ala Tyr Gly Ala Leu Phe Asn Asn Pro Val
                200                 205                 210
Glu Gly Phe Leu Leu Asp Thr Leu Gly Thr Gly Ile Ala Met Thr
                215                 220                 225
Leu Thr His Leu Thr His Arg Glu Gln Ile Ile Leu Phe Thr Phe
                230                 235                 240
Ala Thr Met Lys Thr Val Asp Asp His Cys Gly Tyr Ala Leu Pro
                245                 250                 255
Leu Asp Pro Phe Gln Trp Leu Phe Pro Asn Asn Ala Val Tyr His
                260                 265                 270
Asp Ile His His Gln Gln Phe Gly Ile Lys Thr Asn Phe Ala Gln
                275                 280                 285
Pro Phe Phe Thr Phe Trp Asp Asn Leu Phe Gln Thr Asn Phe Lys
                290                 295                 300
Gly Phe Glu Glu Tyr Gln Lys Lys Gln Arg Arg Val Thr Ile Asp
                305                 310                 315
Lys Tyr Lys Glu Phe Leu Gln Glu Arg Glu Leu Glu Lys Lys Glu
                320                 325                 330
Lys Leu Lys Asn Phe Lys Ala Met Asn Ala Ala Glu Asn Glu Val
                335                 340                 345
Lys Lys Glu Lys
349

<210> 7
<211> 1155
<212> DNA
<213> Saccharomyces cerevisiae
<400> 7
atgtcgacta atacttccaa gactttggaa ctgttttcaa aaaagacggt acaagaacac 60
aatactgcca atgactgctg ggtcacttat caaaacagaa agatttatga cgtgaccagg 120
tttttgagcg aacaccctgg tggtgacgag tccatcttgg actatgctgg taaggacatt 180
actgagatca tgaaagactc agatgtgcat gaacacagcg actccgcgta tgaaatcctt 240
gaggacgaat atttgattgg ttacttggca actgacgaag aggcagcgag attgttgact 300
aacaagaacc ataaggttga agtgcagttg tcagctgacg gtactgagtt tgactccact 360
acttttgtaa aggagttgcc cgccgaggag aaactaagta ttgctacgga ctacagtaac 420
gactacaaaa agcataaatt tttggatctg aaccgtcctt tgctgatgca gattctgcgt 480
agtgatttca agaaagattt ttacgttgac caaatccata gaccaagaca ttacggtaag 540
gggtctgccc cgctatttgg taatttcttg gaaccattaa ctaaaacagc ttggtgggtt 600
gttccagttg cttggttgcc tgtagttgtg taccacatgg gtgttgcttt gaagaacatg 660
aaccagctat ttgcatgttt cttgttctgt gtcggtgtct ttgtttggac tttgattgaa 720
tacggtcttc accgtttcct atttcatttc gatgattggt tacctgaaag taacatcgca 780
ttcgccacac attttctact acatggttgc catcattact tgcccatgga caagtaccgt 840
ttagttatgc cacctactct gttcgtcatc ctttgtgctc catttacaa gttggtattt 900
gctctgctgc cactttattg ggcttacgct ggttttgctg gcggtctttt cggttatgtc 960
tgttatgacg aatgtcattt cttcttgcac cactctaaat tgcctccctt catgcgtaag 1020
ttgaaaaaat atcacctgga acatcattat aaaaactacc aactgggatt tggcgtcaca 1080
tcctggtttt gggacgaagt ttttggcacc tacttaggcc ccgatgcccc attgtccaaa 1140
atgaaatatg aataa 1155

<210> 8
<211> 384
<212> PRT
<213> Saccharomyces cerevisiae
<400> 8
Met Ser Thr Asn Thr Ser Lys Thr Leu Glu Leu Phe Ser Lys Lys
1               5                   10                  15
Thr Val Gln Glu His Asn Thr Ala Asn Asp Cys Trp Val Thr Tyr
                20                  25                  30
Gln Asn Arg Lys Ile Tyr Asp Val Thr Arg Phe Leu Ser Glu His
                35                  40                  45
Pro Gly Gly Asp Glu Ser Ile Leu Asp Tyr Ala Gly Lys Asp Ile
                50                  55                  60
Thr Glu Ile Met Lys Asp Ser Asp Val His Glu His Ser Asp Ser
                65                  70                  75
Ala Tyr Glu Ile Leu Glu Asp Glu Tyr Leu Ile Gly Tyr Leu Ala
                80                  85                  90
Thr Asp Glu Glu Ala Ala Arg Leu Leu Thr Asn Lys Asn His Lys
                95                  100                 105
Val Glu Val Gln Leu Ser Ala Asp Gly Thr Glu Phe Asp Ser Thr
                110                 115                 120

Thr Phe Val Lys Glu Leu Pro Ala Glu Glu Lys Leu Ser Ile Ala
                125                 130                 135
Thr Asp Tyr Ser Asn Asp Tyr Lys Lys His Lys Phe Leu Asp Leu
                140                 145                 150
Asn Arg Pro Leu Leu Met Gln Ile Leu Arg Ser Asp Phe Lys Lys
                155                 160                 165
Asp Phe Tyr Val Asp Gln Ile His Arg Pro Arg His Tyr Gly Lys
                170                 175                 180
Gly Ser Ala Pro Leu Phe Gly Asn Phe Leu Glu Pro Leu Thr Lys
                185                 190                 195
Thr Ala Trp Trp Val Val Pro Val Ala Trp Leu Pro Val Val Val
                200                 205                 210
Tyr His Met Gly Val Ala Leu Lys Asn Met Asn Gln Leu Phe Ala
                215                 220                 225
Cys Phe Leu Phe Cys Val Gly Val Phe Val Trp Thr Leu Ile Glu
                230                 235                 240
Tyr Gly Leu His Arg Phe Leu Phe His Phe Asp Asp Trp Leu Pro
                245                 250                 255
Glu Ser Asn Ile Ala Phe Ala Thr His Phe Leu Leu His Gly Cys
                260                 265                 270
His His Tyr Leu Pro Met Asp Lys Tyr Arg Leu Val Met Pro Pro
                275                 280                 285
Thr Leu Phe Val Ile Leu Cys Ala Pro Phe Tyr Lys Leu Val Phe
                290                 295                 300
Ala Leu Leu Pro Leu Tyr Trp Ala Tyr Ala Gly Phe Ala Gly Gly
                305                 310                 315
Leu Phe Gly Tyr Val Cys Tyr Asp Glu Cys His Phe Phe Leu His
                320                 325                 330
His Ser Lys Leu Pro Pro Phe Met Arg Lys Leu Lys Lys Tyr His
                335                 340                 345
Leu Glu His His Tyr Lys Asn Tyr Gln Leu Gly Phe Gly Val Thr
                350                 355                 360
Ser Trp Phe Trp Asp Glu Val Phe Gly Thr Tyr Leu Gly Pro Asp
                365                 370                 375
Ala Pro Leu Ser Lys Met Lys Tyr Glu
                380                 384

<210>  9
<211>  954
<212>  DNA
<213>  Saccharomyces cerevisiae
<400>  9
atgctgttca gctggcctta tccagaagcc ccgattgaag gttattgggg caagccaact      60
tctctgattg attggtgcga ggagaattat gtcgtatccc cctatattgc agaatggtca     120
aatactatta ccaatagtat attcttaatg accgccttct attctacata tagcgcttgg     180
cgtaataagt tagaaacaag gtatatattg ataggaatgg ggttctcgct ggttggtatt     240
ggttcgtggt tatttcatat gactttacag tatcgttatc aattgctaga cgaactacca     300
atgctgtatg cgaccatcat cccatcgtgg agtatttttg cagaaactca agaaatcttg     360
attaaggatg agaagaaaag gaaggaaagc tcatttagaa tccaaatggt catttctttt     420
atcatgtgtg gtatagtcac cattttaacc tggatttacg ttgtcgtcca aaagccagca     480
attttccaag tcctttatgg tatattgacg cttctagttg tggttctttc tggctggctg     540
acctactatc acgttcatga ttcatttgca aagaaaaatc tttttattac tatggttatg     600
ggcatgattc cttttgtcat tgggttcatt tgctggcaac tagatattca cctgtgttct     660
ttttggatct atatccggag aacatatttg gccctgccat taggtgttct attggaactg     720
catgcttggt ggcatctttt gaccggtact ggtgtctata tcttcgtcgt gtatttgcaa     780
tatttgagaa tattaaccca tggaaatcca aatgacttct tatttatatg gaggtgggga     840
tttttccctg agctggtaag aaagggctta ccgattggta cttcttattc actggagtat     900
ctggggccaa ttgtaaatac acaggtagat gatgaaacaa aaaagaataa ctaa     954

<210>  10
<211>  317
<212>  PRT
<213>  Saccharomyces cerevisiae
<400>  10
Met Leu Phe Ser Trp Pro Tyr Pro Glu Ala Pro Ile Glu Gly Tyr
  1               5                  10                  15
Trp Gly Lys Pro Thr Ser Leu Ile Asp Trp Cys Glu Glu Asn Tyr
                 20                  25                  30
Val Val Ser Pro Tyr Ile Ala Glu Trp Ser Asn Thr Ile Thr Asn
                 35                  40                  45
Ser Ile Phe Leu Met Thr Ala Phe Tyr Ser Thr Tyr Ser Ala Trp
                 50                  55                  60
Arg Asn Lys Leu Glu Thr Arg Tyr Ile Leu Ile Gly Met Gly Phe
                 65                  70                  75
Ser Leu Val Gly Ile Gly Ser Trp Leu Phe His Met Thr Leu Gln
                 80                  85                  90

Tyr Arg Tyr Gln Leu Leu Asp Glu Leu Pro Met Leu Tyr Ala Thr
                95                  100                 105
Ile Ile Pro Ser Trp Ser Ile Phe Ala Glu Thr Gln Glu Ile Leu
                110                 115                 120
Ile Lys Asp Glu Lys Lys Arg Lys Glu Ser Ser Phe Arg Ile Gln
                125                 130                 135
Met Val Ile Ser Phe Ile Met Cys Gly Ile Val Thr Ile Leu Thr
                140                 145                 150
Trp Ile Tyr Val Val Val Gln Lys Pro Ala Ile Phe Gln Val Leu
                155                 160                 165
Tyr Gly Ile Leu Thr Leu Leu Val Val Val Leu Ser Gly Trp Leu
                170                 175                 180
Thr Tyr Tyr His Val His Asp Ser Phe Ala Lys Lys Asn Leu Phe
                185                 190                 195
Ile Thr Met Val Met Gly Met Ile Pro Phe Val Ile Gly Phe Ile
                200                 205                 210
Cys Trp Gln Leu Asp Ile His Leu Cys Ser Phe Trp Ile Tyr Ile
                215                 220                 225
Arg Arg Thr Tyr Leu Ala Leu Pro Leu Gly Val Leu Leu Glu Leu
                230                 235                 240
His Ala Trp Trp His Leu Leu Thr Gly Thr Gly Val Tyr Ile Phe
                245                 250                 255
Val Val Tyr Leu Gln Tyr Leu Arg Ile Leu Thr His Gly Asn Pro
                260                 265                 270
Asn Asp Phe Leu Phe Ile Trp Arg Trp Gly Phe Phe Pro Glu Leu
                275                 280                 285
Val Arg Lys Gly Leu Pro Ile Gly Thr Ser Tyr Ser Leu Glu Tyr
                290                 295                 300
Leu Gly Pro Ile Val Asn Thr Gln Val Asp Asp Glu Thr Lys Lys
                305                 310                 315
Asn Asn
    317

<210>  11
<211>  44
<212>  DNA
<213>  Artificial sequence
<220>
<223>  Designed oligonucleotide as a primer for PCR amplification
<400>  11
ccttctctag aggatccatg gggagccgcg tctcgcggga agac    44

<210>  12
<211>  45
<212>  DNA
<213>  Artificial sequence
<220>
<223>  Designed oligonucleotide as a primer for PCR amplification
<400>  12
ccttcgaatt ccccggccca ggggagcttc tgagcatcac tggtc    45

<210>  13
<211>  25
<212>  DNA
<213>  Artificial sequence
<220>
<223>  Designed oligonucleotide as a primer for PCR amplification
<400>  13
atgtacaaca gaaaagacag agatg    25

<210>  14
<211>  30
<212>  DNA
<213>  Artificial sequence
<220>
<223>  Designed oligonucleotide as a primer for PCR amplification
<400>  14

aaggtacctc atttctcgct caagaaagtt    30

<210>  15
<211>  65
<212>  DNA
<213>  Artificial sequence
<220>
<223>  Designed oligonucleotide as a primer for PCR amplification

<400> 15
ctccggcttc tgcggttttt cttagtcttt ccgcaccaat tttcacagga attcccgggg
atccg    65

<210> 16
<211> 68
<212> DNA
<213> Artificial sequence
<220>
<223> Designed oligonucleotide as a primer for PCR amplification
<400> 16
ggataataaa tacaaacgtg ggaagtcgga gacattgcct ttacccagca agctagcttg
gctgcagg    68

<210> 17
<211> 31
<212> DNA
<213> Artificial sequence
<220>
<223> Designed oligonucleotide as a primer for PCR amplification
<400> 17
ctccggcttc tgcggttttt cttagtcttt c    31

<210> 18
<211> 28
<212> DNA
<213> Artificial sequence
<220>
<223> Designed oligonucleotide as a primer for PCR amplification
<400> 18
ggaagtcgga gacattgcct ttacccag    28

<210> 19
<211> 27
<212> DNA
<213> Artificial sequence
<220>
<223> Designed oligonucleotide as a primer for PCR amplification
<400> 19
cgaattcagc cgaaaacagt cttgctt    27

<210> 20
<211> 36
<212> DNA
<213> Artificial sequence
<220>
<223> Designed oligonucleotide as a primer for PCR amplification
<400> 20
acgaggctgg gatccgctta ccaccgcttt tagtgc    36

<210> 21
<211> 36
<212> DNA
<213> Artificial sequence
<220>
<223> Designed oligonucleotide as a primer for PCR amplification
<400> 21
ggtggtaagc ggatcccagc ctcgtccaaa attgtc    36

<210> 22
<211> 27
<212> DNA
<213> Artificial sequence
<220>
<223> Designed oligonucleotide as a primer for PCR amplification
<400> 22
cgaattcttg ccaacctgat ctgtgaa    27

<210> 23
<211> 26
<212> DNA
<213> Artificial sequence
<220>
<223> Designed oligonucleotide as a primer for PCR amplification
<400> 23
cgaattcccc agaggcaaag atgtta    26

<210> 24
<211> 39
<212> DNA
<213> Artificial sequence
<220>
<223> Designed oligonucleotide as a primer for PCR amplification
<400> 24
tggatggcac ggatccgaaa ggcacacctg tcattatgg 39

<210> 25
<211> 34
<212> DNA
<213> Artificial sequence
<220>
<223> Designed oligonucleotide as a primer for PCR amplification
<400> 25
tgtgcctttc ggatccgtgc catccatttg aatc 34

<210> 26
<211> 30
<212> DNA
<213> Artificial sequence
<220>
<223> Designed oligonucleotide as a primer for PCR amplification
<400> 26
cgaattcctt ttatgatggg agtaactgct 30

<210> 27
<211> 30
<212> DNA
<213> Artificial sequence
<220>
<223> Designed oligonucleotide as a primer for PCR amplification
<400> 27

ctccatgtcg cttaccaccg cttttagtgc 30

<210> 28
<211> 31
<212> DNA
<213> Artificial sequence
<220>
<223> Designed oligonucleotide as a primer for PCR amplification
<400> 28
cgctatactg cagcctcgtc caaaattgtc a 31

<210> 29
<211> 30
<212> DNA
<213> Artificial sequence
<220>
<223> Designed oligonucleotide as a primer for PCR amplification
<400> 29
gtggtaagcg acatggaggc ccagaatacc 30

<210> 30
<211> 31
<212> DNA
<213> Artificial sequence
<220>
<223> Designed oligonucleotide as a primer for PCR amplification
<400> 30
gacgaggctg cagtatagcg accagcattc a 31

<210> 31
<211> 20
<212> DNA
<213> Artificial sequence
<220>
<223> Designed oligonucleotide as a primer for PCR amplification
<400> 31
tccagcacca tctctccttt 20

<210> 32
<211> 20

<212> DNA
<213> Artificial sequence
<220>
<223> Designed oligonucleotide as a primer for PCR amplification
<400> 32
agtgggtcta caccgaccag

## Description

### TECHNICAL FIELD

The present application claims priority based on Japanese Patent Application No. 2005-351366 filed on December 5, 2005.

The present invention relates to methods for producing human ceramide in yeast cells.

### BACKGROUND ART

A tissue known as stratum corneum exists on the outermost layer of the skin, which has a moisturizing function for retaining moisture as well as a barrier function for protecting the skin against external stimulation. The stratum corneum consists of keratinocytes, natural moisturizing factors and intercellular lipids, among which ceramides account for approximately one-half of the total intercellular lipids and play a crucial role for these functions. For example, a common characteristic of atopic dermatitis and senile xerosis is a significant deterioration of moisturizing ability, which is known to mainly result from decreased ceramide levels due to lipid metabolic enzyme abnormalities. Ceramides have also been shown to enhance barrier function, provide whitening effect and inhibit melanogenesis. Ceramides can be externally supplied.

J Invest Dermatol. 96:523-526, 1991 (Non-patent document 1) and Arch Dermatol Res. 283:219-223, 1991 (Non-patent document 2) disclose "decreased ceramide levels in atopic dermatitis and senile xerosis"; J Dermatol Sci. 1:79-83, 1990 (Non-patent document 3) and Acta Derm Venereol. 74:337-340, 1994 (Non-patent document 4) disclose "decreased ceramide levels and lipid metabolic enzyme abnormalities"; Contact Dermatitis. 45:280-285, 2001 (Non-patent document 5) and J Eur Acad Dermatol Venereol. 16:587-594, 2002 (Non-patent document 6) disclose "restoration of barrier function by ceramides"; and Cell Signal 14:779-785, 2002 (Non-patent document 7) discloses "inhibition of melanogenesis by ceramides".

Recently, ceramides have attracted great attention for use in medicines for skin diseases associated with dry sensitive skin or in cosmetics or health and/or /cosmeceutical foods. In fact, a number of products such as cosmetics and food or supplements containing ceramides have already been commercialized, and the market for ceramide materials is continuing to grow.

Ceramide materials of animal origin such as cow were conventionally used, but are currently replaced by those of plant origin such as rice, wheat, soybean and potato because of problems of infections. A recent basic study (J. Clin. Invest. 112:1372-1382, 2003 (Non-patent document 8)) showed the importance of the structures of ceramides in the moisturizing and barrier functions of the skin, which raised questions about whether plant ceramides structurally different from human ceramides are highly functional lipids. Moreover, ceramides are present in animals and plants in minute amounts and are difficult to extract and purify, thus incurring low productivity and high cost, and therefore, it is highly desirable to develop a new production technique capable of overcoming these problems.

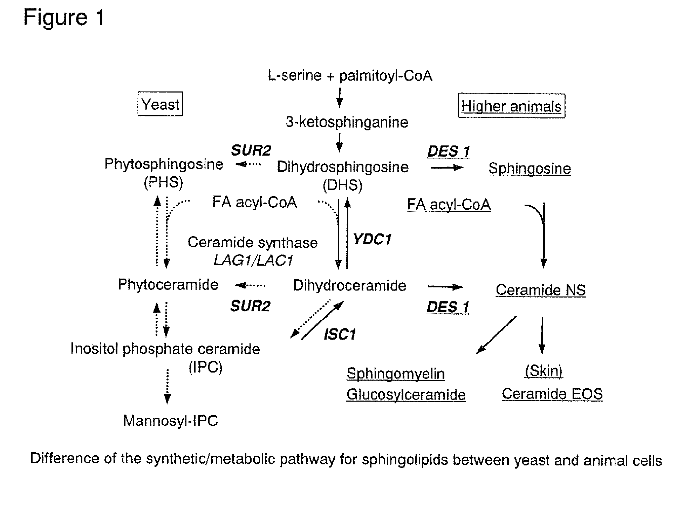

As shown in Figure 1, it is known that dihydrosphingosine (DHS) biosynthesis and the subsequent reactions in the synthetic/metabolic pathway for sphingolipids widely differ between higher animal cells, including human cells and yeast cells. Each enzyme protein involved in various steps in the synthetic/metabolic pathway for sphingolipids and the gene encoding the protein have been known to some degree (Biochemistry. 41:15105-15114. 2002 (Non-patent document 9); J Biol Chem. 277:25512-25518, 2002 (Non-patent document 10); Yeast 9: 267-277, 1993 (Non-patent document 11); J Biol Chem 272:29704-29710, 1997 (Non-patent document 12); J Biol Chem 275:31369-31378, 2000 (Non-patent document 13); J Biol Chem 275:39793-39798, 2000 (Non-patent document 14)).

### References:

Non-patent document 1: J Invest Dermatol. 96:523-526, 1991.
Non-patent document 2: Arch Dermatol Res. 283:219-223, 1991.
Non-patent document 3: J Dermatol Sci. 1:79-83, 1990.
Non-patent document 4: Acta Derm Venereol. 74:337-340, 1994.
Non-patent document 5: Contact Dermatitis. 45:280-285, 2001.
Non-patent document 6: J Eur Acad Dermatol Venereol. 16:587-594, 2002.
Non-patent document 7: Cell Signal 14:779-785, 2002.
Non-patent document 8: J. Clin. Invest. 112:1372-1382, 2003.
Non-patent document 9: Biochemistry. 41:15105-15114, 2002.
Non-patent document 10: J Biol Chem. 277:25512-25518, 2002.
Non-patent document 11: Yeast 9: 267-277, 1993.
Non-patent document 12: J Biol Chem 272:29704-29710, 1997.
Non-patent document 13: J Biol Chem 275:31369-31378, 2000.
Non-patent document 14: J Biol Chem 275:39793-39798, 2000.

### DISCLOSURE OF THE INVENTION

### PROBLEMS TO BE SOLVED BY THE INVENTION

An object of the present invention is to provide a method for producing human ceramide in a yeast cell. The method of the present invention comprises:
1) introducing the sphingoid Δ4-desaturase gene (DES1) by transformation of the yeast cell; and
2) abolishing the expression of the yeast sphinganine C4-hydroxylase gene (SUR2) by transformation of the yeast cell.

In the present invention, the sphingoid Δ4-desaturase gene (DES1) preferably has the amino acid sequence of SEQ ID NO: 2 or an amino acid sequence obtained by deletion, addition or substitution of one or more amino acid residues in SEQ ID NO: 2, and encodes a protein having sphingoid Δ4-desaturase activity.

In the present invention, the yeast sphinganine C4-hydroxylase gene (SUR2) preferably has the amino acid sequence of SEQ ID NO: 6 or an amino acid sequence obtained by deletion, addition or substitution of one or more amino acid residues in SEQ ID NO: 6, and encodes a protein having sphinganine C4-hydroxylase activity.

A preferred embodiment of the method of the present invention further comprises abolishing the expression of the yeast sphingolipid α-hydroxylase gene (SCS7) by transformation of the yeast cell.

A preferred embodiment of the method of the present invention further comprises abolishing the expression of the yeast alkaline dihydroceramidase gene (YDC1) by transformation of the yeast cell.

A preferred embodiment of the method of the present invention further comprises enhancing the expression of the yeast inositol phosphosphingolipid phospholipase C gene (ISC1) by transformation of the yeast cell.

### MEANS TO SOLVE THE PROBLEMS

As a result of careful studies to solve the problems described above, the present inventors have achieved the present invention.

The structures of ceramides depend on the types of enzymes possessed by cells, and vary between species. Yeasts possess no enzyme for synthesizing ceramide NS, which is a main ceramide in higher animals. Thus, necessary enzymes must be introduced into yeast cells to synthesize ceramide NS in yeast cells. Moreover, host cells have a host-derived ceramide synthetic pathway. Therefore, ceramide NS is selectively synthesized with high efficiency by inhibiting the host-derived ceramide synthetic pathway.

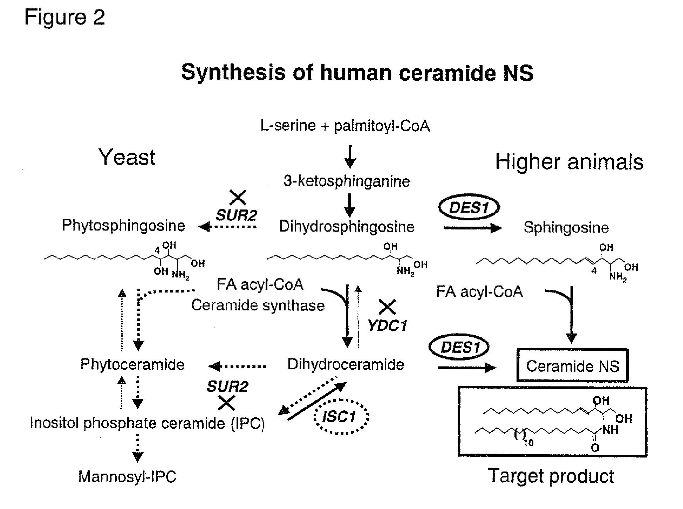

Specifically, in the synthetic/metabolic pathway for sphingolipids, reactions downstream of dihydrosphingosine (DHS) biosynthesis widely differ between higher animal cells including human cells and yeast cells, as shown in Figure 1. More specifically, no human sphingoid base (sphingosine) having a double bond at C-4 of DHS is synthesized in budding yeast (genus Saccharomyces) due to the absence of the sphingoid Δ4-desaturase gene (DES1) (Figure 2). Instead, phytosphingosine (PHS) is synthesized by hydroxylation of C-4 of DHS by an enzyme encoded by the sphinganine C4-hydroxylase gene (SUR2). Then, these sphingoid bases are converted into ceramides.

First, the present inventors thought it important to allow yeast cells to express a sphingoid Δ4-desaturase enzyme not present in yeast cells and to completely or even partially abolish sphinganine C4-hydroxylase enzyme activity in order to produce human ceramide in yeast cells. Thus, we initially 1) prepared the SUR2 gene disruption strain, and 2) introduced the human DES1 gene into the variant strain by transformation of yeast, thereby achieving the present invention. Thus, we first succeeded in producing human ceramide in yeast cells, which had not been achieved before the present invention.

We further optimized the above system to construct a system for efficiently producing human ceramide NS. Specifically, we succeeded in producing human ceramide more efficiently by transformation of yeast with any one of the following 3) to 5):
3) abolishing the expression of the yeast sphingolipid α-hydroxylase gene (SCS7) to prevent hydroxylation of ceramide NS;
4) abolishing the expression of the yeast alkaline dihydroceramidase gene (YDC1); or
5) enhancing the expression of the yeast inositol phosphosphingolipid phospholipase C gene (ISC1); or a combination of 3) - 5).

Thus, the present invention provides methods for conveniently and efficiently producing human ceramide in yeast cells by including 1) and 2) above as essential features, and 3) - 5) as additional features in preferred embodiments.

Accordingly, the present invention relates to methods for producing human ceramide in a yeast cell, comprising:
1) introducing the sphingoid Δ4-desaturase gene (DES1) by transformation of the yeast cell; and
2) abolishing the expression of the yeast sphinganine C4-hydroxylase gene (SUR2) by transformation of the yeast cell.

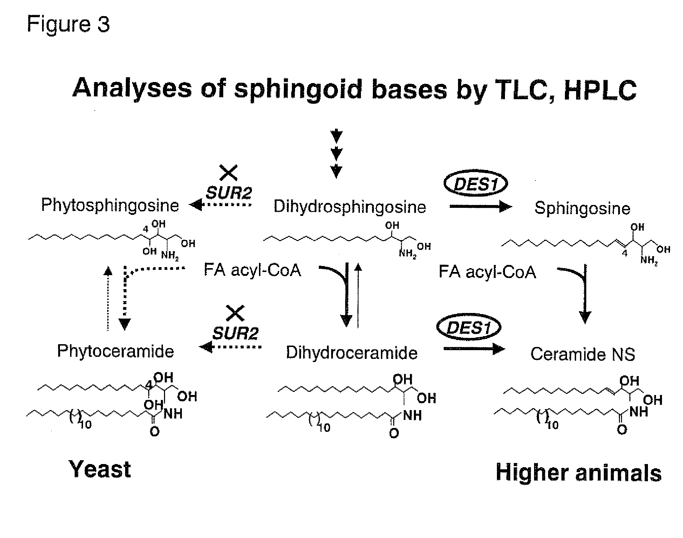

As used herein, human ceramide refers to ceramide NS having the structural formula shown as "target product" in Figure 2, for example. In contrast, phytoceramide is yeast ceramide, which differs from the human ceramide in that the double bond at the 4-position of the human ceramide is substituted by a hydroxy group (Figure 3).

### Sphingoid Δ4-desaturase gene (DES1)

The methods of the present invention comprise introducing the sphingoid Δ4-desaturase gene (DES1) by transformation of the yeast cell, as essential feature 1).

DES1 preferably has, but is not limited to, the amino acid sequence of SEQ ID NO: 2 or an amino acid sequence obtained by deletion, addition or substitution of one or more amino acid residues in SEQ ID NO: 2, and encodes a protein having sphingoid Δ4-desaturase activity.

Genes (nucleic acids) that can be used in the present invention include genomic DNAs (including their corresponding cDNAs), chemically synthesized DNAs, DNAs amplified by PCR, and combinations thereof.

DES1 preferably has the nucleotide sequence of SEQ ID NO: 1. This is a nucleotide sequence encoding a human sphingoid Δ4-desaturase protein having the amino acid sequence of SEQ ID NO: 2, and it is disclosed in, for example, GenBanK^{™}: accession number AF466375.

One or more codons may encode the same amino acid, and this is called degeneracy of the genetic code. Thus, a DNA sequence not completely identical to SEQ ID NO: 1 may encode a protein having an amino acid sequence completely identical to SEQ ID NO: 2. Such a variant DNA sequence may result from silent mutation (e.g., occurring during PCR amplification), or can be the product of deliberate mutagenesis of a native sequence.

DES1 preferably encodes the amino acid sequence of SEQ ID NO: 2. However, it may also have an amino acid sequence obtained by deletion, addition or substitution of one or more amino acid residues. It is intended to encompass any homologous protein so long as it has sphingoid Δ4-desaturase activity. The present invention is not limited to SEQ ID NO: 2 in so far as an amino acid sequence having a comparable function to that of SEQ ID NO: 2 is encoded. "Amino acid change" involves one or more amino acids, preferably 1-20, more preferably 1-10, most preferably 1-5 amino acids.

The amino acid sequence encoded by DES1 has an identity of at least about 70%, preferably about 80% or more, more preferably 90% or more, still more preferably 95% or more, and most preferably 98% or more to the amino acid sequence of SEQ ID NO: 2.

The percent amino acid identity may be determined by visual inspection and mathematical calculation. Alternatively, the percent identity of two protein sequences can be determined by comparing sequence information using the GAP computer program, based on the algorithm of Needleman, S.B. and Wunsch, C.D. (J. Mol. Biol., 48: 443-453, 1970), available from the University of Wisconsin Genetics Computer Group (UWGCG). The preferred default parameters for the GAP program include: (1) a scoring matrix, blosum62, as described by Henikoff, S and Henikoff, J.G. (Proc. Natl. Acad. Sci. USA, 89: 10915-10919, 1992); (2) a gap weight of 12; (3) a gap length weight of 4; and (4) no penalty for end gaps.

Other programs used by those skilled in the art of sequence comparison may also be used. The percent identity can be determined by comparing sequence information using the BLAST program described by Altschul et al. (Nucl. Acids. Res. 25, pp. 3389-3402, 1997), for example. This program is available at the website of National Center for Biotechnology Information (NCBI) or DNA Data Bank of Japan (DDBJ) on the Internet. Various conditions (parameters) for homology searches with the BLAST program are described in detail on the site, and searches are normally performed with default values, though some settings may be appropriately changed.

In the methods of the present invention, sphingoid Δ4-desaturase preferably has the amino acid sequence of SEQ ID NO: 2 or an amino acid sequence at least 70% identical to SEQ ID NO: 2, and sphingoid Δ4-desaturase activity.

It is well known to those skilled in the art that even proteins having the same function may have different amino acid sequences depending on the varieties from which they are derived. DES1 may include such homologs and variants of the nucleotide sequence of SEQ ID NO: 1 so long as they have sphingoid Δ4-desaturase activity. In addition to the human sphingoid Δ4-desaturase protein of SEQ ID NO: 2, the presence of genes encoding proteins showing a similar activity is known in, for example, mouse (M. musculus), drosophila (D. melanogaster), nematode (C. elegans), fission yeast (Schizosaccharomyces pombe), etc. (Non-patent document 10).

The expression "having sphingoid Δ4-desaturase activity" refers to the activity of introducing a double bond into C-4 of dihydrosphingosine to synthesize sphingosine, as shown in Figure 2 or Figure 3. Alternatively, it refers to the activity of introducing a double bond into C-4 of dihydroceramide to synthesize ceramide NS. Sphingosine and/or ceramide NS that are not synthesized in the natural metabolic pathway of yeast are synthesized in transformant yeast cells by introducing DES1.

A preferred sphingoid Δ4-desaturase gene of the present invention also includes a nucleic acid capable of hybridizing to the nucleotide sequence of SEQ ID NO: 1 under stringent conditions; e.g., under conditions of moderate or high stringency and having sphingoid Δ4-desaturase activity.

The expression "under stringent conditions" refers to hybridization under conditions of moderate or high stringency. Specifically, conditions of moderate stringency can be readily determined by those having ordinary skill in the art based on, for example, the length of the DNA. The basic conditions are set forth by Sambrook et al. Molecular Cloning: A Laboratory Manual, 3rd Ed., Chapters 6-7, Cold Spring Harbor Laboratory Press, 2001, and include use of a prewashing solution for the nitrocellulose filters 5 x SSC, 0.5% SDS, 1.0 mM EDTA (pH 8.0), hybridization conditions of about 50% formamide, 2 x SSC to 6 x SSC at about 40°C to 50°C (or other similar hybridization solution, such as Stark's solution, in about 50% formamide at about 42°C), and washing conditions of about 40°C to 60°C, 0.5 to 6 x SSC, 0.1% SDS. Preferably, conditions of moderate stringency include hybridization conditions (and washing conditions) of 6 x SSC at about 50°C. Conditions of high stringency can also be readily determined by the skilled artisan based on, for example, the length of the DNA

Generally, such conditions include hybridization and/or washing at higher temperatures and/or lower salt concentrations than in the conditions of moderate stringency (e.g., hybridization in 6 x SSC to 0.2 x SSC, preferably 6 x SSC, more preferably 2 x SSC, most preferably 0.2 x SSC at about 65°C), and are defined to involve hybridization conditions as above and washing in 0.2 x SSC, 0.1% SDS at about 65°C to 68°C. SSPE (1 x SSPE = 0.15 M NaCl, 10 mM NaH₂PO₄, and 1.25 mM EDTA, pH 7.4) can be substituted for SSC (1 x SSC = 0.15 M NaCl and 15 mM sodium citrate) for use as hybridization and washing buffers, and washing is continued for 15 minutes after completion of hybridization.

As known by those skilled in the art and as further described below, it should be understood that the washing temperature and the washing salt concentration can be adjusted as desired to achieve a desirable degree of stringency by applying basic principles governing hybridization reaction and duplex stability (see e.g., Sambrook et al., 2001). When a nucleic acid is to be hybridized to a target nucleic acid of an unknown sequence, the length of the hybrid is assumed to be that of the nucleic acid to be hybridized. When nucleic acids of known sequences are to be hybridized, the length of the hybrid can be determined by aligning the sequences of the nucleic acids and identifying a single or multiple region(s) having optimal sequence complementarity. The hybridization temperature of a hybrid estimated to have a length of less than 50 bp must be 5-25°C lower than the melting temperature (Tₘ) of the hybrid, where Tₘ is determined by the equation below. For hybrids having a length of less than 18 bp, Tₘ (°C) = 2 (the number of A+T bases) + 4 (the number of G+C bases). For hybrids having a length of18 bp or more, Tₘ = 81.5°C + 16.6 (log₁₀[Na⁺]) + 41 (mole fraction [G+C]) - 0.63 (% formamide) - 500/n, where N is the number of bases in the hybrid, and [Na⁺] is the sodium ion concentration in the hybridization buffer ([Na⁺] in 1 x SSC = 0.165 M) . Preferably, such hybridizing nucleic acids each have a length of at least 8 nucleotides (or more preferably at least 15 nucleotides, or at least 20 nucleotides, or at least 25 nucleotides, or at least 30 nucleotides, or at least 40 nucleotides, or most preferably at least 50 nucleotides), or a length of at least 1% (more preferably at least 25%, or at least 50%, or at least 70%, and most preferably at least 80%) of the length of a nucleic acid to which it hybridizes, and has a sequence identity of at least 50% (more preferably at least 70%, at least 75%, at least 80%, at least 85%, at least 90%, at least 95%, at least 97.5%, or at least 99%, and most preferably at least 99.5%) to a nucleic acid to which it hybridizes. The sequence identity here is determined by comparing the sequences of the nucleic acids to be hybridized when aligned so as to maximize overlap and identity while minimizing sequence gaps, as described in detail above.

The percent nucleic acid identity can be determined by visual inspection and mathematical calculation. Alternatively, the percent identity of two nucleic acid sequences can be determined by visual inspection and mathematical calculation, or more preferably, the comparison is made by comparing sequence information using a computer program. An exemplary, preferred computer program is the Genetics Computer Group (GCG; Madison, Wis.) Wisconsin package version 10.0 program, "GAP" (Devereux et al., 1984, Nucl. Acids Res. 12: 387). This "GAP" program can be used to compare not only two nucleic acid sequences but also two amino acid sequences or a nucleic acid sequence and an amino acid sequence. The preferred default parameters for the "GAP" program include (1) The GCG implementation of a unary comparison matrix (containing a value of 1 for identities and 0 for non-identities) for nucleotides, and the weighted amino acid comparison matrix of Gribskov and Burgess, Nucl. Acids Res. 14: 6745, 1986 as described by Schwartz and Dayhoff, eds., "Atlas of Polypeptide Sequence and Structure", National Biomedical Research Foundation, pp. 353-358, 1979; or other comparable comparison matrices; (2) a penalty of 30 for each gap and an additional penalty of 1 for each symbol in each gap for amino acid sequences, or penalty of 50 for each gap and an additional penalty of 3 for each symbol in each gap for nucleotide sequences; (3) no penalty for end gaps; and (4) no maximum penalty for long gaps. Other programs used by those skilled in the art of sequence comparison can also be used, such as, for example, the BLASTN program version 2.2.7, available for use via the National Library of Medicine website: http://www.ncbi.nlm.nih.gov/blast/bl2seq/bls.ht ml, or the UW-BLAST 2.0 algorithm. Standard default parameter settings for UW-BLAST 2.0 are described at the following Internet site: http://blast.wustl.edu. In addition, the BLAST algorithm uses the BLOSUM62 amino acid scoring matix, and optional parameters that can be used are as follows: (A) inclusion of a filter to mask segments of the query sequence that have low compositional complexity (as determined by the SEG program of Wootton and Federhen (Computers and Chemistry, 1993); also see Wootton and Federhen, 1996, Analysis of compositionally biased regions in sequence databases, Methods Enzymol. 266: 554-71) or segments consisting of short-periodicity internal repeats (as determined by the XNU program of Claverie and States (Computers and Chemistry, 1993)), and (B) a statistical significance threshold for reporting matches against database sequences, or E-score (the expected probability of matches being found merely by chance, according to the stochastic model of Karlin and Altschul, 1990; if the statistical significance ascribed to a match is greater than this E-score threshold, the match will not be reported.); preferred E-score threshold values are 0.5, or in order of increasing preference, 0.25, 0.1, 0.05, 0.01, 0.001, 0.0001, 1e-5, 1e-10, 1e-15, 1e-20, 1e-25, 1e-30, 1e-40, 1e-50, 1e-75, or 1e-100.

The sphingoid Δ4-desaturase gene (DES1) of the present invention also includes a nucleic acid having a nucleotide sequence different from that of SEQ ID NO: 1 by deletion, addition or substitution of one or more nucleotides, but still encoding a protein having sphingoid Δ4-desaturase activity. The number of nucleotides deleted, inserted or substituted is not limited so long as a protein having sphingoid Δ4-desaturase activity is encoded, but is preferably 1 to several thousands, more preferably 1 to 1,000, more preferably 1 to 500, still more preferably 1 to 200, most preferably 1 to 100.

A given amino acid may be replaced, for example, by a residue having similar physiochemical characteristics. Examples of such conservative substitutions include changes from one aliphatic residue to another, such as changes from one to another of Ile, Val, Leu, or Ala; changes from one polar residue to another, such as Lys to Arg, Glu to Asp, or Gln to Asn; or changes from one aromatic residue to another, such as changes from one to another of Phe, Trp, or Tyr. Other well-known conservative substitutions include, for example, changes between entire regions having similar hydrophobic characteristics. Those skilled in the art can introduce desired deletions, insertions or substitutions by well-known gene engineering techniques using, for example, site-specific mutagenesis as described in Sambrook et al. (2001), supra.

### Yeast sphinganine C4-hydroxylase gene (SUR2)

The methods of the present invention comprise abolishing the expression of the yeast sphinganine C4-hydroxylase gene (SUR2) by transformation of the yeast cell, as essential feature 2).

SUR2 preferably has, but is not limited to, the amino acid sequence of SEQ ID NO: 6 or an amino acid sequence obtained by deletion, addition or substitution of one or more amino acid residues in SEQ ID NO: 6, and encodes a protein having sphinganine C4-hydroxylase activity.

SUR2 preferably has the nucleotide sequence of SEQ ID NO: 5. This is the nucleotide sequence encoding a yeast sphinganine C4-hydroxylase protein having the amino acid sequence of SEQ ID NO: 6, and it is disclosed in, for example, SGD (Saccharomyces Genome Database, http://www.yeastgenome.org/).

SUR2 preferably encodes the amino acid sequence of SEQ ID NO: 6. However, it may also have an amino acid sequence obtained by deletion, addition or substitution of one or more amino acid residues. It is intended to encompass any homologous protein so long as it has sphinganine C4-hydroxylase activity. The present invention is not limited to SEQ ID NO: 6 so long as an amino acid sequence having a comparable function to that of SEQ ID NO: 6 is encoded. "Amino acid change" involves one or more amino acids, preferably 1-20, more preferably 1-10, most preferably 1-5 amino acids.

The expression "having sphinganine C4-hydroxylase activity" refers to the activity of introducing a hydroxyl group into C-4 of dihydrosphingosine to synthesize phytosphingosine, as shown in Figure 2 or Figure 3. Alternatively, it refers to the activity of introducing a hydroxyl group into C-4 of dihydroceramide to synthesize phytoceramide. In the present invention, the synthesis of phytosphingosine and/or phytoceramide that are synthesized in the natural metabolic pathway of yeast is partially or completely inhibited by partially or completely abolishing the expression of SUR2 by transformation of the yeast cell. Sphingosine and/or ceramide NS can be efficiently synthesized by suppressing the expression of the SUR2 gene and expressing the DES1 gene.

The amino acid sequence encoded by SUR2 has an identity of at least about 70%, preferably about 80% or more, more preferably 90% or more, still more preferably 95% or more, most preferably 98% or more to the amino acid sequence of SEQ ID NO: 6.

A preferred yeast sphinganine C4-hydroxylase gene (SUR2) of the present invention also includes a nucleic acid capable of hybridizing to the nucleotide sequence of SEQ ID NO: 5 under stringent conditions; e.g., under conditions of moderate or high stringency and having yeast sphinganine C4-hydroxylase activity.

The yeast sphinganine C4-hydroxylase gene (SUR2) of the present invention also includes a nucleic acid having a nucleotide sequence different from that of SEQ ID NO: 5 by deletion, addition or substitution of one or more nucleotides, but still encoding a protein having sphinganine C4-hydroxylase activity.

Common matters such as "deletion, addition or substitution of amino acids and/or nucleotides", "percent identity of amino acids and/or nucleotides", hybridization "under stringent conditions" are as described above for DES1.

### Yeast sphingolipid α-hydroxylase gene (SCS7)

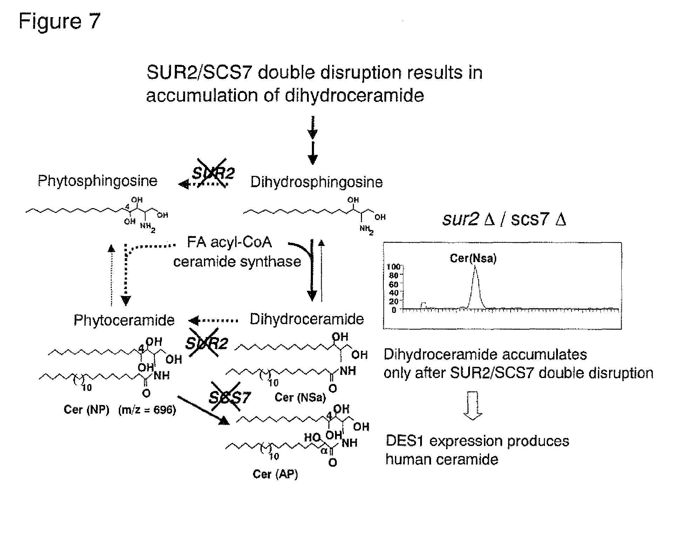

The methods of the present invention may further comprise abolishing the expression of the yeast sphingolipid α-hydroxylase gene (SCS7) by transformation of the yeast cell. SCS7 has the activity of adding a hydroxyl group to the α-carbon of a fatty acid amide-linked to the sphingoid base of phytoceramide, dihydroceramide, and ceramide NS to synthesize Cer(AP), Cer(ASa), and Cer(AS), respectively, in Figure 3 or Figure 7, for example. Even if desired dihydroceramide or ceramide NS is synthesized, it is further hydroxylated by the presence of SCS7. Thus, the present invention preferably comprises abolishing the expression of SCS7.

SCS7 preferably has, but not limited to, the amino acid sequence of SEQ ID NO: 8 or an amino acid sequence obtained by deletion, addition or substitution of one or more amino acid residues in SEQ ID NO: 8, and encodes a protein having sphingolipid α-hydroxylase activity.

SCS7 preferably has the nucleotide sequence of SEQ ID NO: 7. This is the nucleotide sequence encoding a yeast sphingolipid α-hydroxylase protein having the amino acid sequence of SEQ ID NO: 8, and it is disclosed in e.g., SGD (Saccharomyces Genome Database, http://www.yeastgenome.org/).

SCS7 preferably encodes the amino acid sequence of SEQ ID NO: 8. However, it may also have an amino acid sequence obtained by deletion, addition or substitution of one or more amino acid residues. It is intended to encompass any homologous protein so long as it has sphingolipid α-hydroxylase activity. The present invention is not limited to SEQ ID NO: 8 so long as an amino acid sequence having a comparable function to that of SEQ ID NO: 8 is encoded. "Amino acid change" involves one or more amino acids, preferably 1-20, more preferably 1-10, most preferably 1-5 amino acids.

The expression "having sphingolipid α-hydroxylase activity" refers to the activity of adding a hydroxyl group to the α-carbon of a fatty acid amide-linked to the sphingoid base of phytoceramide, dihydroceramide, and ceramide NS to synthesize Cer(AP), Cer(ASa), and Cer(AS), respectively, as shown in Figure 7, for example. In the present invention, undesirable hydroxylation of dihydroceramide or ceramide NS is inhibited by partially or completely abolishing the expression of SCS7, preferably by transformation of the yeast cell.

The amino acid sequence encoded by SCS7 has an identity of at least about 70%, preferably about 80% or more, more preferably 90% or more, still more preferably 95% or more, most preferably 98% or more to the amino acid sequence of SEQ ID NO: 8.

A preferred yeast sphingolipid α-hydroxylase gene (SCS7) of the present invention also includes a nucleic acid capable of hybridizing to the nucleotide sequence of SEQ ID NO: 7 under stringent conditions, e.g., under conditions of moderate or high stringency and having yeast sphingolipid α-hydroxylase activity.

The yeast sphingolipid α-hydroxylase gene (SCS7) of the present invention also includes a nucleic acid having a nucleotide sequence different from that of SEQ ID NO: 7 by deletion, addition or substitution of one or more nucleotides, but still encoding a protein having sphingolipid α-hydroxylase activity.

Common matters such as "deletion, addition or substitution of amino acids and/or nucleotides", "percent identity of amino acids and/or nucleotides", hybridization "under stringent conditions" are as described above for DES1.

### Yeast alkaline dihydroceramidase gene (YDC1)

The methods of the present invention may further comprise abolishing the expression of the yeast alkaline dihydroceramidase gene (YDC1) by transformation of the yeast cell.

YDC1 encodes a protein having the activity of degrading dihydroceramide to synthesize dihydrosphingosine, as shown in Figure 1 - Figure 3, for example. YDC1 activity promotes so to speak a reverse reaction to ceramide synthesis to reduce dihydroceramide as a precursor for ceramide NS synthesis. Thus, the present invention preferably comprises abolishing the expression of YDC1.

YDC1 preferably has, but is not limited to, the amino acid sequence of SEQ ID NO: 10 or an amino acid sequence obtained by deletion, addition or substitution of one or more amino acid residues in SEQ ID NO: 10, and encodes a protein having alkaline dihydroceramidase activity.

YDC1 preferably has the nucleotide sequence of SEQ ID NO: 9. This is the nucleotide sequence encoding a yeast alkaline dihydroceramidase protein having the amino acid sequence of SEQ ID NO: 10, and it is disclosed in, for example, SGD (Saccharomyces Genome Database, http://www.yeastgenome.org/).

YDC1 preferably encodes the amino acid sequence of SEQ ID NO: 10. However, it may also have an amino acid sequence obtained by deletion, addition or substitution of one or more amino acid residues. It is intended to encompass any homologous protein so long as it has alkaline dihydroceramidase activity. The present invention is not limited to SEQ ID NO: 10 so long as an amino acid sequence having a comparable function to that of SEQ ID NO: 10 is encoded. "Amino acid change" involves one or more amino acids, preferably 1-20, more preferably 1-10, and most preferably 1-5 amino acids.

The expression "having alkaline dihydroceramidase activity" refers to the activity of degrading dihydroceramide to synthesize dihydrosphingosine, as shown in Figure 1 - Figure 3, for example. In the present invention, undesirable degradation of dihydroceramide is inhibited by partially or completely abolishing the expression of YDC1, preferably by transformation of the yeast cell.

The amino acid sequence encoded by YDC1 has an identity of at least about 70%, preferably about 80% or more, more preferably 90% or more, still more preferably 95% or more, most preferably 98% or more to the amino acid sequence of SEQ ID NO: 10.

A preferred yeast alkaline dihydroceramidase gene (YDC1) of the present invention also includes a nucleic acid capable of hybridizing to the nucleotide sequence of SEQ ID NO: 9 under stringent conditions, e.g., under conditions of moderate or high stringency and having yeast alkaline dihydroceramidase activity.

The yeast alkaline dihydroceramidase gene (YDC1) of the present invention also includes a nucleic acid having a nucleotide sequence different from that of SEQ ID NO: 9 by deletion, addition or substitution of one or more nucleotides, but still encoding a protein having alkaline dihydroceramidase activity.

Common matters such as "deletion, addition or substitution of amino acids and/or nucleotides", "percent identity of amino acids and/or nucleotides", hybridization "under stringent conditions" are as described above for DES1.

### Yeast inositol phosphosphingolipid phospholipase C gene (ISC1)

The methods of the present invention may further comprise enhancing the expression of the yeast inositol phosphosphingolipid phospholipase C gene (ISC1) by transformation of the yeast cell.

ISC1 encodes a protein having the activity of synthesizing dihydroceramide from inositol phosphate ceramide (IPC) as shown in Figure 1 and Figure 2, for example. ISC1 activity increases dihydroceramide as a precursor for ceramide NS synthesis. ISC1 is a gene naturally occurring in yeast cells, but the synthesis of human ceramide can be promoted by introducing this gene to enhance its expression by transformation of the yeast cell.

ISC1 preferably has, but is not limited to, the amino acid sequence of SEQ ID NO: 4 or an amino acid sequence obtained by deletion, addition or substitution of one or more amino acid residues in SEQ ID NO: 4, and encodes a protein having inositol phosphosphingolipid phospholipase C activity.

ISC1 preferably has the nucleotide sequence of SEQ ID NO: 3. This is the nucleotide sequence encoding a yeast inositol phasphosphingolipid phospholipase C protein having the amino acid sequence of SEQ ID NO: 4, and it is disclosed in, for example, SGD (Saccharomyces Genome Database, http://www.yeastgenome.org/).

ISC1 preferably encodes the amino acid sequence of SEQ ID NO: 4. However, it may also have an amino acid sequence obtained by deletion, addition or substitution of one or more amino acid residues. It is intended to encompass any homologous protein so long as it has inositol phosphosphingolipid phospholipase C activity. The present invention is not limited to SEQ ID NO: 4 so long as an amino acid sequence having a comparable function to that of SEQ ID NO: 4 is encoded. "Amino acid change" involves one or more amino acids, preferably 1-20, more preferably 1-10, and most preferably 1-5 amino acids.

The expression "having inositol phosphosphingolipid phospholipase C activity" refers to the activity of synthesizing dihydroceramide from inositol phosphate ceramide (IPC), as shown in Figure 1 and Figure 2. In the present invention, dihydroceramide as a precursor for ceramide NS synthesis is increased to promote the synthesis of human ceramide by enhancing the expression of ISC1, preferably by transformation of the yeast cell.

The amino acid sequence encoded by ISC1 has an identity of at least about 70%, preferably about 80% or more, more preferably 90% or more, still more preferably 95% or more, and most preferably 98% or more to the amino acid sequence of SEQ ID NO: 4.

A preferred yeast inositol phosphosphingolipid phospholipase C gene (ISC1) of the present invention also includes a nucleic acid capable of hybridizing to the nucleotide sequence of SEQ ID NO: 3 under stringent conditions, e.g., under conditions of moderate or high stringency and having yeast inositol phosphosphingolipid phospholipase C activity.

The yeast inositol phosphosphingolipid phospholipase C gene (ISC1) of the present invention also includes a nucleic acid having a nucleotide sequence different from that of SEQ ID NO: 3 by deletion, addition or substitution of one or more nucleotides, but still encoding a protein having inositol phosphosphingolipid phospholipase C activity.

Common matters such as "deletion, addition or substitution of amino acids and/or nucleotides", "percent identity of amino acids and/or nucleotides", hybridization "under stringent conditions" are as described above for DES1.

In a more preferred embodiment, the present invention provides a method for producing human ceramide in a yeast cell, comprising:
1) introducing the sphingoid Δ4-desaturase gene (DES1) by transformation of the yeast cell;
2) abolishing the expression of the yeast sphinganine C4-hydroxylase gene (SUR2) by transformation of the yeast cell;
3) abolishing the expression of the yeast sphingolipid α-hydroxylase gene (SCS7) by transformation of the yeast cell;
4) abolishing the expression of the yeast alkaline dihydroceramidase gene (YDC1) by transformation of the yeast cell; and
5) enhancing the expression of the yeast inositol phosphosphingolipid phospholipase C gene (ISC1) by transformation of the yeast cell.

### Methods for introducing and expressing genes by transformation of yeast

In the present invention, the expression of DES1 and the enhanced expression of ISC1 in the yeast cell can be performed by any known method. Preferably, the method comprises transforming a host yeast cell with an expression vector containing DES1 and/or ISC1, and cultivating the transformant yeast cell under conditions allowing the expression of the nucleic acid.

The yeast species that can be used in the methods of the present invention are preferably, but are not limited to, yeast species of the genus Saccharomyces. Saccharomyces cerevisiae, Saccharomyces pastorianus, Saccharomyces bayanus, and Saccharomyces kluyveri are more preferred. Budding yeast species including the genus Saccharomyces have been analyzed most extensively for ceramide synthesis and metabolism at the genetic level. Thus, they can be used to rapidly optimize the methods for producing human ceramide according to the present invention. Moreover, yeast cells are easy to culture and have been traditionally used for food manufacturing. In addition, they can be used to establish a method for extracting/purifying large amounts of ceramides conveniently, safely and inexpensively.

In the present invention, known yeast expression vectors can be used to introduce and express genes. In the examples below, known gene expression vectors for yeast pRS series (p4XX) (Mumberg et al., Gene, 156, 119, 1995) and pYE22m (Ashikari et al., Appl Microbiol Biotechnol, 30, 515, 1989) were used.

Any of multicopy (YEp), single copy (YCp), and chromosome integration (YIp) vectors can be used for introduction into yeast. For example, a number of expression vectors for yeast are known to those skilled in the art and can be used in the methods of the present invention, including YEp vectors such as YEp24 (J. R. Broach et al., Experimental Manipulation of Gene Expression, Academic Press, New York, 83, 1983), YCp vectors such as YCp50 (M. D. Rose et al., gene, 60, 237, 1987), and YIp vectors such as YIp5 (K. Struhl et al., Proc. Natl. Acad. Sci. USA, 76, 1035, 1979).

In addition to each gene of interest, expression vectors can typically contain a selectable marker and an origin of replication for proliferation in host cells. Vectors also optionally contain a transcription or translation regulatory sequence preferably derived from yeast fused to a nucleic acid of the present invention.

Examples of regulatory sequences include transcriptional promoters, operators, or enhancers, an mRNA ribosomal binding site, and appropriate sequences which control the initiation and termination of transcription and translation. Nucleotide sequences are operably linked to a regulatory sequence when the regulatory sequence is functionally associated with the DNA sequences. Thus, a promoter nucleotide sequence is operably linked to a DNA sequence if the promoter nucleotide sequence controls the transcription of the DNA sequence. An origin of replication that confers the ability to replicate in a host cell, and a selection gene by which transformants are identified are generally incorporated into expression vectors. As for selectable markers, those commonly used can be routinely used. Examples are genes resistant to antibiotics such as tetracycline, ampicillin, kanamycin, neomycin, hygromycin or spectinomycin and auxotrophic genes such as HIS3, TRP1.

Yeast vectors will often contain an origin of replication sequence derived from the 2µ yeast plasmid, an autonomous replication sequence (ARS), a promoter region, a sequence for polyadenylation, a sequence for transcription termination, and a selectable marker gene.

Vectors can be conveniently prepared by routine fusion of a desired gene to a recombination vector available in the art (e.g. plasmid DNA). Methods for integrating a DNA fragment of a gene into a vector such as a plasmid are described in, for example, Sambrook, J., and Russell, D.W. (2001). Molecular Cloning: A Laboratory Manual, 3rd ed. (New York: Cold Spring Harbor Laboratory Press). Commercially available ligation kits (e.g. available from TAKARA) can be conveniently used.

Methods for introducing a plasmid into a host cell include calcium phosphate or calcium chloride/rubidium chloride transfection, electroporation, electroinjection, chemical treatment with PEG or the like, the use of a gene gun, etc. described in Sambrook, J. et al. (2001) (supra.).

### Methods for abolishing each gene by transformation of yeast

The methods of the present invention comprise abolishing the expression of the yeast sphinganine C4-hydroxylase gene (SUR2) by transformation of the yeast cell.

In preferred embodiments, the methods of the present invention further comprise either one or a combination of:
abolishing the expression of the yeast sphingolipid α-hydroxylase gene (SCS7) by transformation of the yeast cell; and
abolishing the expression of the yeast alkaline dihydroceramidase gene (YDC1) by transformation of the yeast cell.

As used herein, the expression "abolishing the expression of each gene" means that the protein activity encoded by each gene is not produced. Any abolishment is included in the scope of the present invention to achieve the purposes in the methods of the present invention, so long as the protein activity encoded by the gene is not exerted eventually, such as disrupting the gene on the genome of a yeast cell, inhibiting the transcription of the gene, inhibiting the translation of the gene into a protein, or inhibiting activity even if it is translated into a protein. Abolishment may be partial or complete. Typically, each gene on the genome of a mother cell is disrupted to partially or completely delete the gene.

The expression of the SUR2, SCS7 and YDC1 genes can be abolished by known methods.

For example, a DNA fragment containing upstream and downstream nucleotide sequences of each gene fused to a selectable marker was used to delete the gene by homologous recombination with the natural genome sequence of yeast in the examples herein below.

Disruption of a gene can be performed by addition or deletion of one or more nucleotides in a region responsible for the expression of a gene product in the target gene, such as a coding region or a promoter region, or by entire deletion of these regions. Such methods for gene disruption can be found in known publcations (e.g., see Yeast 10, 1793 (1994), Yeast 15, 1541 (1999), Proc. Natl. Acad. Sci. USA, 76, 4951 (1979), Methods in Enzymology, 101, 202 (1983), etc.).

In addition to gene disruption, other methods for suppressing the expression of each gene for the purposes of the present invention include antisense methods (e.g., see Hirajima and Inoue: New Biochemistry Experiment Course 2 Nucleic acid, IV. Gene Replication and Expression (Japanese Biochemical Society Ed., Tokyo Kagaku Dozin Co., Ltd.) pp.319-347, 1993, etc.), RNAi methods (see Domestic announcement No. 2002-516062 of PCT application; US Patent Laid-Open Publication No. 2002/086356A; Nature Genetics, 24 (2), 180-183, 2000, etc.), ribozyme methods (see FEBS Lett. 228: 228, 1988; FEBS Lett. 239: 285, 1988; Nucl. Acids. Res. 17: 7059, 1989, etc.), cosuppression (e.g., see Smyth DR: Curr. Biol. 7: R793, 1997, Martienssen R: Curr. Biol. 6: 810, 1996, etc.), etc.

### Methods for verifying ceramide synthesis

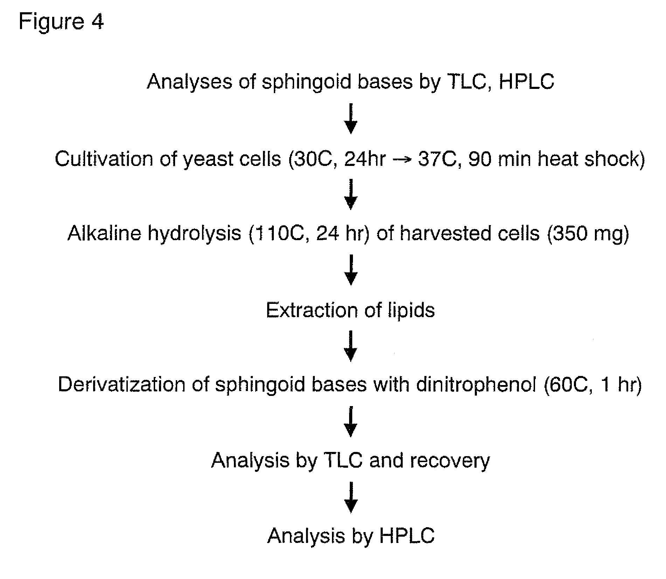

The human ceramide (ceramide NS) produced by the methods of the present invention can be extracted/purified by using known methods. The methods of the present invention allow large-scale culture and convenient and rapid extraction/purification of the ceramide because yeast cells are used. One of the preferred embodiments for extraction/purification used in the examples is shown in Figure 4.

The purified ceramide can be identified by using known methods for analyzing sphingoid bases. Analytical methods include e.g., TLC, HPLC, mass spectrometry (e.g., LC-MS, LC-MS/MS, FT-MS), etc.

### ADVANTAGES OF THE INVENTION

According to the methods for producing ceramide using yeast transformants of the present invention, human ceramide highly functional on the human skin can be inexpensively produced.

### BRIEF EXPLANATION OF THE DRAWINGS

[Fig. 1] Figure 1 shows the synthetic/metabolic pathways for sphingolipids in yeast and higher animal cells.
[Fig. 2] Figure 2 shows an overview of a preferred embodiment of a method for producing human ceramide in yeast cells according to the present invention.
[Fig. 3] Figure 3 shows the molecular species of sphingoid bases and ceramides and the structural formulae thereof in yeast and higher animals.
[Fig. 4] Figure 4 schematically shows steps of the present invention from cultivation of yeast cells to analyses by TLC and HPLC.
[Fig. 5] Figure 5 shows the results of the identification of the molecular species of ceramides by LC-MS analysis in a yeast SUR2/SCS7 double disruption strain expressing the human DES1 gene.

Left panels show from top to bottom:
a total cation chromatogram of the ceramide region of the yeast SUR2/SCS7 double disruption strain expressing the human DES1 gene;
a chromatogram generated only at m/z 680 (peak RT13.35) in the same strain;
a chromatogram generated only at m/z 678 (peak RT13.69) in the same strain; and
a cation chromatogram of standard ceramide NS.
Right panels show from top to bottom:
a mass spectrum at a peak of m/z 680; and
a mass spectrum at a peak of m/z 678.
It could be verified that ceramide NS (Cer(NS) (Mw 678)) had been synthesized in the yeast SUR2/SCS7 double disruption strain expressing the human DES1 gene.

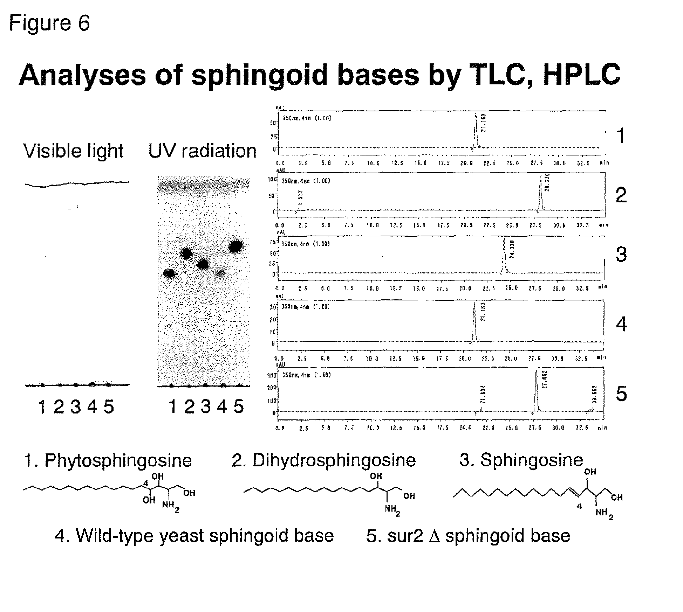

[Fig. 6] Figure 6 shows the results of analyses of sphingoid bases by TLC and HPLC in a SUR2 disruption strain.

Sample 1: phytosphingosine standard,
Sample 2: dihydrosphingosine standard,
Sample 3: sphingosine standard,
Sample 4: sphingoid base extracted from wild-type yeast,
Sample 5: sphingoid base extracted from the SUR2 disruption strain.
Panels show from left to right:
   TLC observed under visible light (DNP-derivatized sphingoid bases are observed as yellow spots),
   TLC observed under UV radiation (DNP-derivatized sphingoid bases are observed as dark blue spots), and
   HPLC chromatograms.

The sphingoid base extracted from the SUR2 disruption strain (sample 5) was identified as dihydrosphingosine from the location of the HPLC peak agreeing with that of sample 2. In contrast, the sphingoid base extracted from wild-type yeast (sample 4) was identified as phytosphingosine from the location of the HPLC peak agreeing with that of sample 1.
[Fig. 7] Figure 7 shows the results of LC-MS analysis of a SUR2/SCS7 double disruption strain (ion chromatogram at m/z 500-800). In the SUR2/SCS7 double disruption strain, accumulation of dihydroceramide (Cer(NSa)) was verified. If DES1 is expressed by this disruption strain, human ceramide will be synthesized.

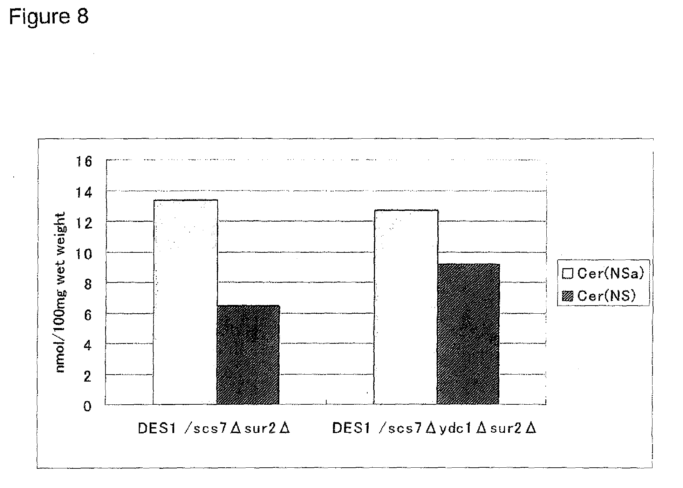

[Fig. 8] Figure 8 shows ceramide levels in the yeast SUR2/SCS7 double disruption strain expressing human DES1 and the yeast SUR2/SCS7/YDC1 triple disruption strain expressing human DES1.

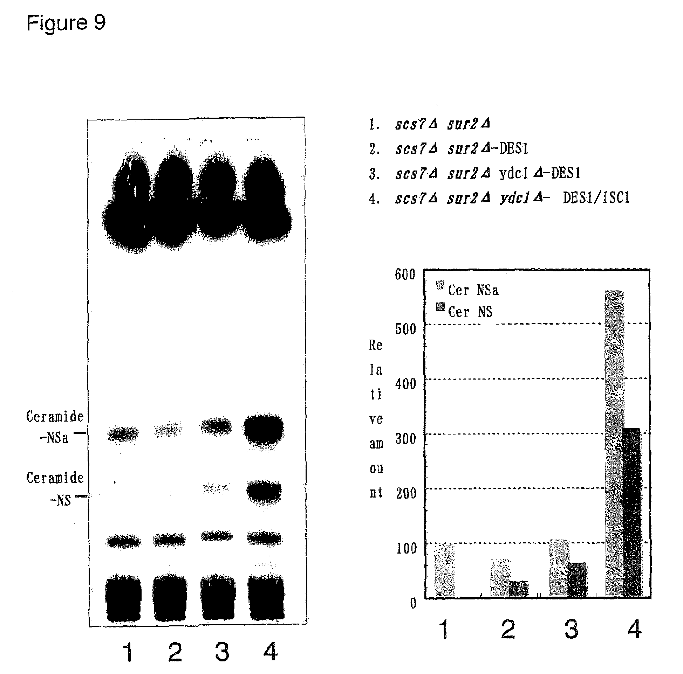

[Fig. 9] Figure 9 shows the results of TLC analysis of yeast ceramides using tritiated (³H) D-erythro-dihydrosphingosine and the results of quantification of radioactively labeled ceramides using a Bioimage Analyzer (BAS). The amount of ceramide NS synthesized increased in order from left: sample 1: SUR2/SCS7 double disruption strain; sample 2: yeast SUR2/SCS7 double disruption strain expressing the human DES1 gene; sample 3: yeast SUR2/SCS7/YDC1 triple disruption strain expressing the human DES1; and sample 4: yeast SUR2/SCS7/YDC1 triple disruption strain expressing human DES1 and highly expressing ISC1.

### EXAMPLES

The following examples further illustrate the present invention but are not intended to limit the technical scope of the invention. Those skilled in the art can readily add modifications/changes to the present invention in the light of the description herein, and those modifications/changes are included in the technical scope of the present invention.

### [Example 1]

### Preparation of a vector expressing the human sphingoid Δ4-desaturase gene (DES1)

Based on the nucleotide sequence of the human sphingoid Δ4-desaturase gene (DES1) in a public database (GenBanK^{™}: accession number AF466375) (SEQ ID NO: 1), primers des1f (SEQ ID NO: 11) and des1R (SEQ ID NO: 12) were prepared.

SEQ ID NO: 11:
   5'-CCTTCTCTAGAGGATCCATGGGGAGCCGCGTCTCGCGGGAAGAC-3'
SEQ ID NO: 12:
   5'-CCTTCGAATTCCCCGGGCCAGGGGAGCTTCTGAGCATCACTGGTC-3'.
The primer pair was used to perform PCR with a human cDNA library as a template. The resulting PCR product (about 1.1 kb) was cloned into the gene expression vector for yeast pKO11 (Kamei et al., J. Biol. Chem., 273, 28341, 1998; provided by Dr. K. Tanaka) using BamHI and SmaI sites.

The nucleotide sequence of the clone was determined by the Sanger method to confirm that it was identical to the sequence in the database. The clone was subcloned into the gene expression vector for yeast pRS series (p4XX) (Mumberg et al., Gene, 156, 119, 1995) using BamHI and Xho1 sites.

### [Example 2]

### Preparation of a vector expressing the yeast (Saccharomyces cerevisiae) inositol phosphosphingolipid phospholipase C gene (ISC1)

Based on the nucleotide sequence of the yeast (Saccharomyces cerevisiae) inositol phosphosphingolipid phospholipase C gene (ISC1) in a public yeast genome database (SGD (Saccharomyces Genome Database, http://www.yeastgenome.org/)) (SEQ ID NO: 3), primers isc1F (SEQ ID NO: 13) and isc1R (SEQ ID NO: 14) were prepared.

SEQ ID NO: 13: 5'-ATGTACAACAGAAAAGACAGAGATG-3'
SEQ ID NO: 14: 5'-AAGGTACCTCATTTCTCGCTCAAGAAAGTT-3'.
The primer pair was used to perform PCR with a routinely prepared yeast genomic DNA as a template. The resulting PCR product (about 1.4 kb) was cloned into the pCR-BluntII-TOPO vector (Invitrogen), and the nucleotide sequence of the clone was determined by the Sanger method (F. Sanger, Science, 214, 1215, 1981) to confirm that it was identical to the sequence in the database.

The clone was subcloned into the gene expression vector for yeast pYE22m (Ashikari et al., Appl Microbiol Biotechnol, 30, 515, 1989) using EcoRI and KpnI sites.

### [Example 3]

### Preparation of a disruption strain of the yeast sphinganine C4-hydroxylase gene (SUR2)

Based on the nucleotide sequence of the yeast sphinganine C4-hydroxylase gene (SUR2) in a public yeast genome database (SGD (Saccharomyces Genome Database, http://www.yeastgenome.org/)) (SEQ ID NO: 5), primers sur2F (SEQ ID NO: 15) and sur2R (SEQ ID NO: 16) were prepared.

SEQ ID NO: 15:
   5'- CTCCGGCTTCTGCGGTTTTTCTTAGTCTTTCCGCACCAATTTTCACAGGAATTCCCGGGGAT CCGG-3'
SEQ ID NO: 16:
   5'- GGATAATAAATACAAACGTGGGAAGTCGGAGACATTGCCTTTACCCAGCAAGCTAGCTTGGC TGCAGG-3'.
The primer pair was used to perform PCR with the plasmid pYDp-L (Berben et al., Yeast, 7, 475, 1991) as a template, thereby giving a PCR product containing a 295-bp upstream region of the SUR2 gene, a selectable marker and a 75-bp downstream region of the SUR2 gene fused together. This PCR product was transformed into the strain FK113 (MATa, ura3, his3, leu2, lys2, trp1, barl-1), and the transformants were selected in an auxotrophic medium to give a SUR2 gene disruption strain.

The disruption of the SUR2 gene was confirmed by PCR using confirmation primers designed to be amplified into fragments of different lengths depending on whether the gene is normal or disrupted (SEQ ID NOs: 17 and 18).

SEQ ID NO: 17: 5'-CTCCGGCTTCTGCGGTTTTTCTTAGTCTTTC-3'
SEQ ID NO: 18: 5'-GGAAGTCGGAGACATTGCCTTTACCCAG-3'.

### [Example 4]

### Preparation of a double disruption strain of the yeast SUR2 and yeast sphingolipid α-hydroxylase (SCS7) genes

Based on the nucleotide sequence of the yeast sphingolipid α-hydroxylase gene (SCS7) in a public yeast genome database (SGD (Saccharomyces Genome Database, http://www.yeastgenome.org/)) (SEQ ID NO: 7), primers scs7up280F (SEQ ID NO: 19) and scs7up280R (SEQ ID NO: 20), and scs7down280F (SEQ ID NO: 21) and scs7down280R (SEQ ID NO: 22) were prepared.

SEQ ID NO: 19: 5'-CGAATTCAGCCGAAAACAGTCTTGCTT-3'
SEQ ID NO: 20: 5'- ACGAGGCTGGGATCCGCTTACCACCGCTTTTAGTGC-3'
SEQ ID NO: 21: 5'- GGTGGTAAGCGGATCCCAGCCTCGTCCAAAATTGTC-3'
SEQ ID NO: 22: 5'-CGAATTCTTGCCAACCTGATCTGTGAA-3'.
The primer pairs were used to perform PCR with a routinely prepared yeast genomic DNA as a template to give PCR products corresponding to an upstream region of about 280 bp and a downstream region of about 280 bp of the SCS7 gene, respectively. The primers scs7up280R and scs7down280F have complementary sequences and contain a BamHI site at the boundary between the upstream and downstream regions of the SCS7 gene. The PCR products corresponding to the upstream and downstream regions of about 280bp of the SCS7 gene were mixed to perform PCR using the primers scs7up280F and scs7down280R, thereby giving a PCR product containing the upstream and downstream regions of the SCS7 gene flanking the BamHI site. This product was cloned into a vector obtained by self-ligation of pUC19 that had been digested with BamHI and then blunt-ended with T4 DNA polymerase, using EcoRI sites contained in the primers scs7up280F and scs7down280R. The nucleotide sequence of the clone was determined by the Sanger method (F. Sanger, Science, 214, 1215, 1981) to confirm that it was identical to the sequences of the upstream and downstream regions of SCS7 in the database.

The plasmid pYDp-U (Berben et al., Yeast, 7, 475, 1991) was digested with BamHI, and a fragment of about 1.1 kb containing the URA3 gene was inserted into the BamHI site of the plasmid containing the upstream and downstream regions of the SCS7 gene.

A fragment obtained by digesting the resulting plasmid containing the URA3 gene flanked by the upstream and downstream regions of the YDC1 gene with EcoRI was routinely transformed into the SUR2 gene disruption strain obtained in Example 3 above. Screening on complete minimal plate lacking uracil (SC-Ura) gave a SUR2/SCS7 double disruption strain. To allow for the subsequent plasmid introduction, the SUR2/SCS7 double disruption strain was plated on minimal plate containing 0.1% 5-fluoroorotic acid to select a strain having lost uracil auxotrophy.

### [Example 5]

### Preparation of a triple disruption strain of the yeast SUR2, yeast alkaline dihydroceramidase (YDC1), and SCS7 genes

Based on the nucleotide sequence of the yeast alkaline dihydroceramidase gene (YDC1) in a public yeast genome database (SGD (Saccharomyces Genome Database, http://www.yeastgenorne.arg/)) (SEQ ID NO: 9), primers ydclup280F (SEQ ID NO: 23) and ydc1up280R (SEQ ID NO: 24), and ydcldown250F (SEQ ID NO: 25) and ydcldown250R (SEQ ID NO: 26) were prepared.

SEQ ID NO: 23: 5'-CGAATTCCCCAGAGGCAAAGATGTTA-3'
SEQ ID NO: 24: 5'-TGGATGGCACGGATCCGAAAGGCACACCTGTCATTATGG-3'
SEQ ID NO: 25: 5'-TGTGCCTTTCGGATCCGTGCCATCCATTTGAATC-3'
SEQ ID NO: 26: 5'-CGAATTCCTTTTATGATGGGAGTAACTGCT-3'.
The primer pairs were used to perform PCR with a routinely prepared yeast genomic DNA as a template, thereby giving PCR products corresponding to an upstream region of about 280 bp and a downstream region of about 250 bp of the YDC1 gene, respectively. The primers ydc1up280R and ydcldown250F have complementary sequences and contain a BamHI site at the boundary between the upstream and downstream regions of the YDC1 gene.

The PCR products corresponding to the upstream region of about 280 bp and downstream region of about 250 bp of the YDC1 gene were mixed to perform PCR using the primers ydc1up280F and ydc1down250R, thereby giving a PCR product containing the upstream and downstream regions of the YDC1 gene flanking the BamHI site. This product was cloned into a vector obtained by self-ligation of pUC19 that had been digested with BamHI and then blunt-ended with T4 DNA polymerase, by use of EcoRI sites contained in the primers ydc1up280F and ydc1down250R. The nucleotide sequence of the clone was determined by the Sanger method (F. Sanger, Science, 214, 1215, 1981) to confirm that it was identical to the sequences of the upstream and downstream regions of YDC1 in the database.

The plasmid pYDp-H (Berben et al., Yeast, 7, 475, 1991) was digested with BamHI, and a fragment of about 1.2 kb containing the HIS3 gene was inserted into the BamHI site of the plasmid containing the upstream and downstream regions of the YDC1 gene.

A fragment obtained by digesting the resulting plasmid containing the HIS3 gene flanked by the upstream and downstream regions of the YDC1 gene with EcoRI was routinely transformed into the SUR2 gene disruption strain. Screening on complete minimal plate lacking histidine (SC-His) gave a SUR2/YDC1 double disruption strain.

Based on the nucleotide sequence of the yeast sphingolipid α-hydroxylase (SCS7) in a public yeast genome database (SGD (Saccharomyces Genome Database, http://www.yeastgenome.arg/)) (SEQ ID NO: 7), primers scs7up280F (SEQ ID NO: 19) and scs7up280R_G418 (SEQ ID NO: 27), and scs7down280F_G418 (SEQ ID NO: 28) and scs7down280R (SEQ ID NO: 22) were then prepared.

SEQ ID NO: 19: 5'-CGAATTCAGCCGAAAACAGTCTTGCTT-3'
SEQ ID NO: 27: 5'-CTCCATGTCGCTTACCACCGCTTTTAGTGC-3'
SEQ ID NO: 28: 5'-CGCTATACTGCAGCCTCGTCCAAAATTGTCA -3'
SEQ ID NO: 22: 5'-CGAATTCTTGCCAACCTGATCTGTGAA-3'.
The primer pairs were used to perform PCR with a routinely prepared yeast genomic DNA as a template, thereby giving PCR products corresponding to an upstream region of about 280 bp and a downstream region of about 280 bp of the SCS7 gene, respectively.

Primers G418PCR2F (SEQ ID NO: 29) and G418PCR2R (SEQ ID NO: 30) were also prepared and used to perform PCR with the plasmid pFA6a-kanMX4 (EMBL AJ002680) as a template, thereby giving a PCR product containing a kanamycin and geneticin (G418) resistance gene.

SEQ ID NO: 29: 5'-GTGGTAAGCGACATGGAGGCCCAGAATACC-3'
SEQ ID NO: 30: 5'-GACGAGGCTGCAGTATAGCGACCAGCATTCA-3'.
The primers scs7up280R_G418 (SEQ ID NO: 27) and G418PCR2F (SEQ ID NO: 29), and the primers scs7down280F_G418 (SEQ ID NO: 28) and G418PCR2R (SEQ ID NO: 30) have complementary sequences, and the three PCR products were mixed to perform PCR using the primers scs7up280F and scs7down280R, thereby giving a PCR product containing the 280-bp upstream region of the SCS7 gene, the kanamycin and G418 resistance gene and the 280-bp downstream region of the SCS7 gene fused together. The nucleotide sequence of the clone was determined by the Sanger method (F. Sanger, Science, 214, 1215, 1981) directly using the PCR product as a template to confirm that it was identical to the sequence in the database.

This PCR product was routinely transformed into the SUR2/YDC1 double gene disruption strain, and the transformants were screened on YPD plate containing 300 mg/L G418 (1% yeast extract, 2% polypeptone, 2% glucose, 2% agar) to give a SUR2/YDC1/SCS7 gene triple disruption strain.

### [Example 6]

### Preparation of transformant strains of the yeast ceramide synthetic/metabolic system carrying a human DES1-expressing plasmid

A strain expressing the DES1 gene (Example 1) or a strain expressing the DES1 gene and highly expressing the ISC1 gene (Example 2) was constructed using each gene disruption strain obtained in Examples 3-5 above.

I. SUR2 disruption (Example 3) + human DES1 gene expression (Example 1);
II. SLTR2/SCS7 double disruption (Example 4) + human DES1 gene expression;
III. SUR2/SCS7/YDC1 triple disruption (Example 5) + human DES1 gene expression;
IV. SUR2/SCS7/YDC1 triple disruption (Example 5) + human DES1 gene expression and enhanced expression of ISC1 gene (Examples 1 and 2).
The human DES1-expressing plasmid of Example 1 and the ISC1-expressing plasmid of Example 2 were routinely transformed into the disruption strains obtained in Examples 3-5. Cells transformed with the DES1 gene and cells transformed with the DES1 and ISC1 genes were selected on complete minimal plates lacking uracil and complete minimal plates lacking uracil and tryptophan, respectively (SC-Ura and SC-Ura, Trp, respectively).

The expression of the human DES1 gene in the resulting transformant strains was verified by reverse transcription reaction of total RNA purified by an RNeasy kit (Qiagen) using Superscript II (Invitrogen) with primer Hdes1_R (SEQ ID NO: 31) and PCR (RT-PCR) of the reaction product using Ex-taq (Takara Bio) with Hdes1_F (SEQ ID NO: 32) and Hdes1_R (SEQ ID NO: 31).

SEQ ID NO: 31: 5'-TCCAGCACCATCTCTCCTTT-3'
SEQ ID NO: 32: 5'-AGTGGGTCTACACCGACCAG-3'.
The enhanced expression of the ISC1 gene was also verified by reverse transcription reaction using primer isc1R (SEQ ID NO: 14) and RT-PCR using primers isc1F (SEQ ID NO: 13) and isc1R (SEQ ID NO: 14).

### [Example 7]

### Analyses of sphingoid bases in the transformant strains of the yeast ceramide synthetic/metabolic system carrying a human DES1-expressing plasmid

The yeast SUR2 disruption strain expressing the human DES1 gene (I), the yeast SUR2/SCS7 double disruption strain expressing the human DES1 gene (II), and the yeast SUR2/SCS7/YDC1 triple disruption strain expressing the human DES1 gene (III) obtained in Example 6 were cultivated in liquid minimal medium containing tryptophan, histidine, leucine and lysine (SD+Trp, His, Leu, and Lys), and the yeast SUR2/SCS7/YDC1 triple disruption strain expressing the human DES1 and highly expressing ISC1 (IV) was cultivated in liquid minimal medium containing histidine, leucine and lysine (SD+His, Leu, and Lys) at 30°C for 24 hours. Then, they were incubated under heat shock conditions at 37°C for 90 minutes, and sphingoid bases were extracted from the cells and derivatized with dinitrophenol as described in a publication (Sperling et al., Journal of Biological chemistry, 273, 28590, 1998).

The sphingoid bases were analyzed by thin layer chromatography (TLC) and high-speed liquid chromatography (HPLC). The procedures are briefly described below.

Cells (wet weight 350 mg) were directly hydrolyzed in 3 ml of 1, 4-dioxane/water, 1:1 (v/v) containing 10% (w/v) Ba(OH)₂ at 110°C for 24 hours. Released sphingoid bases were extracted by separation into layers with chloroform/1, 4-dioxane/water, 8:3:8 (v/v/v). The organic layers were washed with equal amounts of 0.1 M KOH and 0.5 M KCl, and then reacted with 0.2 ml of a 0.5% (v/v) solution of 1-fluoro-2,4-dinitrobenzene in methanol and 0.8 ml of 2M borate/KOH (pH 10.5) at 60°C for 30 minutes to derivatize the sphingoid bases with dinitrophenol (DNP-derivatization). After the reaction, the resulting organic layers were dried in vacuo and the resulting DNP-derivatized sphingoid bases were dissolved in chloroform and then developed with chloroform/methanol, 9:1 (v/v) on silica gel 60 TLC plates. The DNP-derivatized sphingoid bases were observed as yellow spots (dark bleu under UV radiation).

Then, the DNP-derivatized sphingoid bases were recovered from the TLC plates and extracted with chloroform/methanol, 2:1 (v/v), and then separated into layers with chloroform/methanol/0.1 M KOH, 2:1:1 (v/v/v). The resulting organic layers were dried in vacuo, and then dissolved in methanol to prepare HPLC samples. HPLC was performed on a silica gel ODS column, eluting with a linear gradient of 80% methanol/acetonitrile/2-propanol (10:3:1, v/v/v) and 20% water to 0% water (flow rate 1 ml/min, 40 min), and UV absorption at 350 nm was monitored.

For easier understanding, a scheme for analyses of sphingoid bases by TLC and HPLC is shown in Figure 4.

The amounts of sphingosine bases in cells were determined on the basis of the HPLC data obtained by similarly analyzing a predetermined amount of a synthetic sphingosine base purchased from Sigma. Table 1 shows the calculated amounts of sphingosine bases accumulated in 100 mg of cells. The amounts of sphingosine bases in cells increased in the order of the yeast SUR2 disruption strain expressing human DES1, the yeast SUR2/SCS7 double disruption strain expressing human DES1, the yeast SUR2/SCS7/YDC1 triple disruption strain expressing human DES1, and the yeast SUR2/SCS7/YDC1 triple disruption strain expressing human DES1 and highly expressing ISC1.

Table 1. Amounts of sphingosine bases in 100 mg of yeast cells

**Table 1**

| | Amounts of sphingosine bases in µg |
|---|---|
| Yeast SUR2 disruption strain expressing human DES1 | 3.98 |
| Yeast SUR2/SCS7 double disruption strain expressing human DES1 | 6.08 |
| Yeast SUR2/SCS7/YDC1 triple disruption strain expressing human DES1 | 8.35 |
| Yeast SUR2/SCS7/YDC1 triple disruption strain expressing human DES1 and highly expressing ISC1 | 9.32 |

### [Example 8]

### Extraction of yeast lipids for LC-MS analysis

The yeast transformants of ceramide synthetic pathway genes of Example 6 were cultivated in 250 ml of liquid minimal medium containing histidine, leucine, lysine, and tryptophan (SD+His, Leu, Lys and Trp, except that tryptophan was not contained for the strain highly expressing ISC1) at 30°C with shaking at 140 rpm for 24 hours, and then incubated under heat shock conditions at 37°C for 90 minutes. The cells were harvested and washed twice with sterilized water, and a suspension of 100 mg of the cells in 500 µl of sterilized water was prepared. The suspension was vigorously stirred with glass beads at room temperature for 5 minutes to disrupt the cells. Lipids were extracted by adding chloroform and methanol in a ratio of chloroform : methanol : suspension of 10:10:3. The extracts were centrifuged and the resulting supernatants were collected and concentrated/dried by blowing with nitrogen gas.

### [Example 9]

### Identification of the molecular species of ceramides by LC-MS analysis

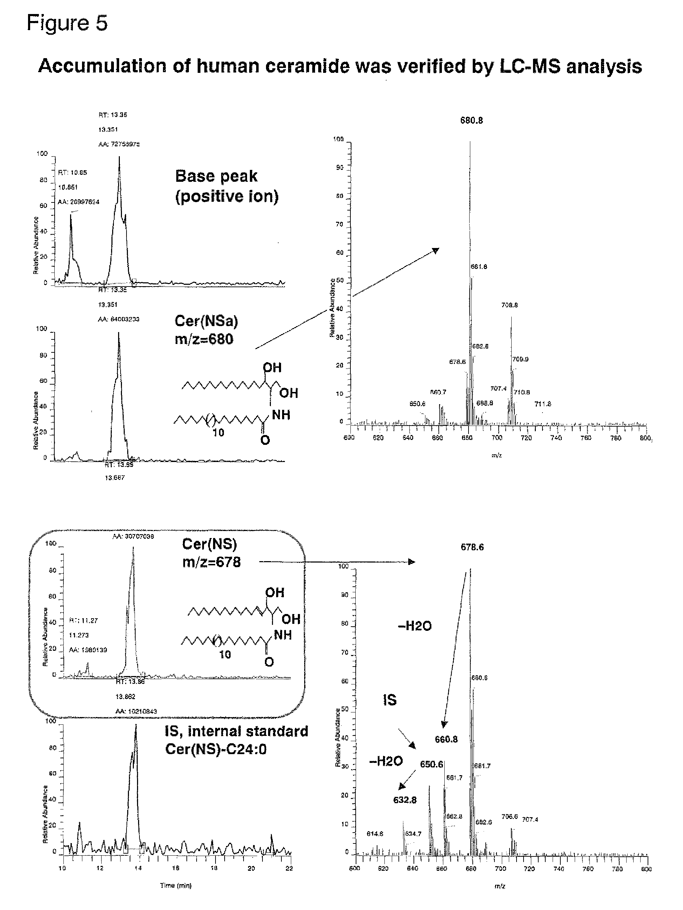

The yeast lipids extracted in Example 8 were extracted with hexane/isopropanol (3:2 v/v), washed with 6.67% aqueous sodium sulfate, and then analyzed by the apparatus described in Japanese Patent Laid-open Publication No. 2003-28849 to identify the molecular species of ceramides. The results of the identification of the molecular species of ceramides in the yeast SUR2/SCS7 double disruption strain expressing the human DES1 gene are shown in Figure 5.

As shown in Figure 5, it was verified that ceramide NS (Cer(NS)) (Mw 678) had been synthesized in the yeast SUR2/SCS7 double disruption strain expressing the human DES1 gene.

It was also verified that ceramide NS (Cer(NS)) (Mw 678) had been synthesized in the yeast SUR2/SCS7/YDC1 triple disruption strain expressing human DES1, and the amounts of ceramide NS (Cer(NS)) accumulated in 100 mg of cells successively increased from 6.5 nmol in the yeast SUR2/SCS7 double disruption strain expressing the human DES1 gene to 9.2 nmol in the yeast SUR2/SCS7/YDC1 triple disruption strain expressing human DES1, as shown in Figure 8.

### [Example 10]

### Analyses of sphingoid bases in the SUR2 disruption strain

The SUR2 disruption strain was analyzed for sphingoid bases by TLC and HPLC. The TLC and HPLC analyses were performed according to the method described in Example 8. The results are shown in Figure 6.

The sphingoid base extracted from the SUR2 disruption strain (sample 5) was identified as dihydrosphingosine from the location of the HPLC peak agreeing with that of sample 2. In contrast, the sphingoid base extracted from wild-type yeast (sample 4) was identified as phytosphingosine from the location of the HPLC peak agreeing with that of sample 1. This shows that dihydrosphingosine is preferentially synthesized in the SUR2 disruption strain in contrast to wild-type yeast.

Sphingosine as a precursor of ceramide NS will be synthesized by introducing and expressing DES1 in this SUR2 disruption strain.

### [Example 11]

### Ceramide analysis in the SUR2/SCS7 disruption strain

The SUR2/SCS7 double disruption strain was analyzed by LC-MS according to the method described in Example 8. The results are shown in Figure 7. In the SUR2/SCS7 double disruption strain, accumulation of dihydroceramide (Cer(NSa)) was demonstrated, rather than a sphingoid base with an amide-linked fatty acid having a hydroxyl group at the α carbon (Cer ASa) or Cer(AP)). Human ceramide will be synthesized by expressing DES1 in this disruption strain.

### [Example 12]

### Ceramide analysis using tritiated (³H) D-erythro-dihydrosphingosine

The yeast transformants of ceramide synthetic pathway genes described above were cultivated in liquid minimal medium containing histidine, leucine, lysine, and tryptophan (SD+His, Leu, Lys and Trp, except that tryptophan was not contained for the strain highly expressing ISC1) at 25°C with shaking at 150 rpm for 24 hours, and then the yeast cells were harvested and suspended in liquid minimal medium to prepare 5 ml of a suspension (OD600=0.5). The suspension was incubated with 10 µl (10 µCi) of tritiated (³H) D-erythro-dihydrosphingosine overnight at 25°C (Zanolari et al., The EMBO Journal, 19, 2824, 2000). The reaction was quenched with 200 µl of 250 mM NaF and 250 mM NaN₃, and then washed three times with ice-cooled sterilized water, and the cells were suspended in 66 µl of sterilized water.

The suspension was vigorously stirred with glass beads to disrupt the cells. Lipids were extracted by adding chloroform and methanol in a ratio of chloroform : methanol : suspension of 10:10:3. The extracts were centrifuged and the resulting supernatants were collected and concentrated/dried by blowing with nitrogen gas. The samples were dissolved in 100 µl of chloroform-methanol-water (10:10:3), and reacted with 20 µl of a 0.6 N solution of NaOH in methanol at 30°C for 90 min, and then neutralized with a 0.6 N acetic acid solution. The reaction solution was desalted by butanol extraction, and the resulting butanol layer (upper layer) was concentrated/dried by blowing with nitrogen gas.

The lipids were dissolved in 20 µl of chloroform-methanol (1:1), spotted on borate-impregnated thin layer chromatography (TLC) plates, and developed with chloroform-methanol (9:1) (Triola et al., Molecular Pharmacology, 66, 1671, 2004). After the development, radioactively labeled ceramides were analyzed by a Bioimage Analyzer (BAS). The results are shown in Figure 9.

If dihydroceramide (CerNSa) in the SUR2/SCS7 double disruption strain is assumed to be 100%, ceramide NS (CerNS) increased in order from 30% in the yeast SUR2/SCS7 double disruption strain expressing the human DES1 gene was, 64% in the yeast SUR2/SCS7/YDC1 triple disruption strain expressing human DES1, and then 309% in the yeast SUR2/SCS7/YDC1 triple disruption strain expressing human DES1 and highly expressing ISC1.

## Claims

1. A method for producing human ceramide in a yeast cell, comprising:
1) introducing the sphingoid Δ4-desaturase gene (DES1) by transformation of the yeast cell; and
2) abolishing the expression of the yeast sphinganine C4-hydroxylase gene (SUR2) by transformation of the yeast cell.

2. The method of claim 1 wherein the sphingoid Δ4-desaturase gene (DES1) has the amino acid sequence of SEQ ID NO: 2 or an amino acid sequence obtained by deletion, addition or substitution of one or more amino acid residues in SEQ ID NO: 2, and encodes a protein having sphingoid Δ4-desaturase activity.

3. The method of claim 1 or 2 wherein the yeast sphinganine C4-hydroxylase gene (SUR2) has the amino acid sequence of SEQ ID NO: 6 or an amino acid sequence obtained by deletion, addition or substitution of one or more amino acid residues in SEQ ID NO: 6, and encodes a protein having sphinganine C4-hydroxylase activity.

4. The method of any one of claims 1 to 3, further comprising abolishing the expression of the yeast sphingolipid α-hydroxylase gene (SCS7) by transformation of the yeast cell.

5. The method of any one of claims 1 to 4 wherein the yeast sphingolipid α-hydroxylase gene has the amino acid sequence of SEQ ID NO: 8 or an amino acid sequence obtained by deletion, addition or substitution of one or more amino acid residues in SEQ ID NO: 8, and encodes a protein having sphingolipid α-hydroxylase activity.

6. The method of any one of claims 1 to 5, further comprising abolishing the expression of the yeast alkaline dihydroceramidase gene (YDC1) by transformation of the yeast cell.

7. The method of any one of claims 1 to 6 wherein the yeast alkaline dihydroceramidase gene (YDC1) has the amino acid sequence of SEQ ID NO: 10 or an amino acid sequence obtained by deletion, addition or substitution of one or more amino acid residues in SEQ ID NO: 10, and encodes a protein having alkaline dihydroceramidase activity.

8. The method of any one of claims 1 to 7, further comprising enhancing the expression of the yeast inositol phosphosphingolipid phospholipase C gene (ISC1) by transformation of the yeast cell.

9. The method of any one of claims 1 to 8 wherein the yeast inositol phosphosphingolipid phospholipase C gene (ISC1) has the amino acid sequence of SEQ ID NO: 4 or an amino acid sequence obtained by deletion, addition or substitution of one or more amino acid residues in SEQ ID NO: 4, and encodes a protein having inositol phosphosphingolipid phospholipase C activity.

10. A method for producing human ceramide in a yeast cell, comprising:
1) introducing the sphingoid Δ4-desaturase gene (DES1) by transformation of the yeast cell;
2) abolishing the expression of the yeast sphinganine C4-hydroxylase gene (SUR2) by transformation of the yeast cell;
3) abolishing the expression of the yeast sphingolipid α-hydroxylase gene (SCS7) by transformation of the yeast cell;
4) abolishing the expression of the yeast alkaline dihydroceramidase gene (YDC1) by transformation of the yeast cell; and
5) enhancing the expression of the yeast inositol phasphosphingolipid phospholipase C gene (ISC1) by transformation of the yeast cell.

11. The method of any one of claims 1 to 10 wherein the yeast is selected from yeast species of the genus Saccharomyces.
